(19) Europäisches Patentamt
European Patent Office
Office européen des brevets

(11) EP 4 480 984 A1

(12) # EUROPEAN PATENT APPLICATION
published in accordance with Art. 153(4) EPC

(43) Date of publication:
**25.12.2024 Bulletin 2024/52**

(21) Application number: **23752629.8**

(22) Date of filing: **19.01.2023**

(51) International Patent Classification (IPC):
***C08G 61/02*** (2006.01) ***C07D 303/48*** (2006.01)
***C08G 59/06*** (2006.01) ***C08G 59/20*** (2006.01)
***C08J 5/04*** (2006.01)

(52) Cooperative Patent Classification (CPC):
**C07D 303/48; C08G 59/06; C08G 59/20; C08G 61/02; C08J 5/04**

(86) International application number:
**PCT/JP2023/001430**

(87) International publication number:
**WO 2023/153160 (17.08.2023 Gazette 2023/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.02.2022 JP 2022020772**

(71) Applicant: **DIC Corporation**
**Tokyo 174-8520 (JP)**

(72) Inventors:
- **ONDA, Shinji**
  **Ichihara-shi, Chiba 290-8585 (JP)**
- **MORINAGA, Kunihiro**
  **Ichihara-shi, Chiba 290-8585 (JP)**

(74) Representative: **TBK**
**Bavariaring 4-6**
**80336 München (DE)**

(54) **PHENOLIC RESIN, EPOXY RESIN, CURABLE RESIN COMPOSITION, CURED PRODUCT, FIBER-REINFORCED COMPOSITE MATERIAL, AND FIBER-REINFORCED RESIN MOLDED ARTICLE**

(57) Provided are a phenolic resin having a specific structure that is used to obtain an epoxy resin having excellent handlingability at low viscosity, the epoxy resin capable of forming a cured product having low water absorption properties and high bending properties (bending strength, bending elastic modulus, bending strain, etc.), a curable resin composition containing the epoxy resin, a cured product obtained from the curable resin composition, a fiber-reinforced composite material, and a fiber-reinforced resin molded article. The phenolic resin is a reaction product of a catechol compound, a phenol compound, and an o-xylylene skeleton-containing compound, and has a catechol skeleton derived from the catechol compound, a phenol skeleton derived from the phenol compound, and an o-xylylene skeleton derived from the o-xylylene skeleton-containing compound.

EP 4 480 984 A1

## Description

Technical Field

The present invention relates to a phenolic resin having a specific structure, an epoxy resin obtained using the phenolic resin, a curable resin composition containing the epoxy resin, a cured product obtained from the curable resin composition, a fiber-reinforced composite material, and a fiber-reinforced resin molded article.

Background Art

An epoxy resin is a curable resin that has an epoxy group in the molecule and can form a cross-linking network with the epoxy group, resulting in curing.

A curable resin composition containing the epoxy resin as an essential component has various uses, and is widely used for a matrix of a fiber-reinforced composite material, a heat-dissipating member, a coating material, a semiconductor, a printed wiring board, and the like due to excellent mechanical strength, heat resistance, water resistance, insulating properties, and the like of a cured product of the curable resin composition.

A fiber-reinforced resin molded article that is reinforced with reinforcement fibers especially has features such as light weight and excellent mechanical strength, to which attention has been paid, and the fiber-reinforced resin molded article is increasingly used for various structures including housings or various members of automobiles, aircrafts, ships, and the like. In particular, the application of a carbon fiber composite material (CFRP) to the aircraft and automobile fields is increased, and therefore the amount of epoxy resin used as a matrix resin thereof is continuously increased.

Currently, examples of an epoxy resin mainly used for the carbon fiber composite material include a diglycidyl ether form of bisphenol A (for example, see PTL 1). The epoxy resins satisfy a constant level of properties required for the carbon fiber composite material, such as elastic modulus, strength, heat resistance, and wet heat resistance.

However, the market requires an epoxy resin that has increased elastic modulus and increased strength and does not decrease elastic modulus and strength even after water absorption. Currently, the physical properties of the epoxy resin do not sufficiently satisfy the requirement of the market.

In particular, as the physical properties of a cured product obtained using the epoxy resin, an increase in elastic modulus is tried, but the strength and elongation (strain) of the cured product are generally decreased. Thus, it is difficult to satisfy both an increase in elastic modulus and other physical properties.

Citation List

Patent Literature

PTL 1: Japanese Unexamined Patent Application Publication No. 2003-201388

Summary of Invention

Technical Problem

An object of the present invention is to provide a phenolic resin having a specific structure that forms an epoxy resin having excellent handlingability at low viscosity, the epoxy resin capable of forming a cured product having low water absorption properties and high bending properties (bending strength, bending elastic modulus, bending strain, etc.), a curable resin composition containing the epoxy resin, a cured product obtained from the curable resin composition, a fiber-reinforced composite material, and a fiber-reinforced resin molded article. Solution to Problem

The present inventors have intensively investigated to achieve the object, and as a result, found that a cured product obtained from a curable resin composition that contains an epoxy resin that is obtained using a phenolic resin having a specific structure and has excellent handlingability at low viscosity expresses low water absorption properties and high bending properties. Thus, the present invention has been completed.

Specifically, the present invention relates to a phenolic resin that is a reaction product of a catechol compound, a phenol compound, and an o-xylylene skeleton-containing compound, and has a catechol skeleton derived from the catechol compound, a phenol skeleton derived from the phenol compound, and an o-xylylene skeleton derived from the o-xylylene skeleton-containing compound.

The phenolic resin of the present invention is preferably represented by the following general formula (1).

[Formula 1]

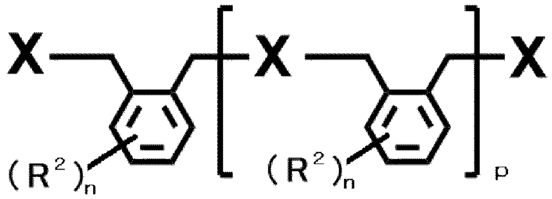

(1)

[Formula 2]

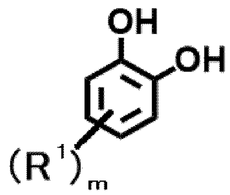

(2)

[Formula 3]

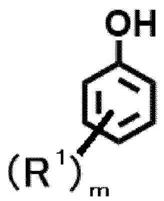

(3)

In the formula (1), X is a catechol compound represented by a formula (2), or a phenol compound represented by a formula (3), $R^1$ is a hydrogen atom, a hydrocarbon group having 1 to 4 carbon atoms, or an alkoxy group having 1 to 4 carbon atoms, $R^2$ is a hydrogen atom or a methyl group, m is an integer of 0 to 3, n is an integer of 0 to 4, and p is an integer of 0 to 50.

The hydroxy group equivalent of the phenolic resin of the present invention is preferably 90 to 140 g/eq.

The present invention relates to an epoxy resin that is a reaction product that has a glycidyl ether group obtained by a reaction of a phenolic hydroxy group of the phenolic resin with epihalohydrin.

The epoxy resin of the present invention is preferably represented by the following general formula (4).

[Formula 4]

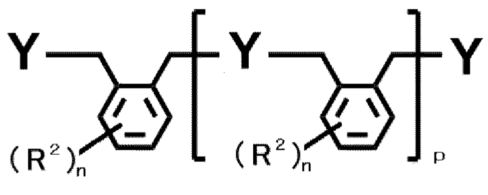

(4)

[Formula 5]

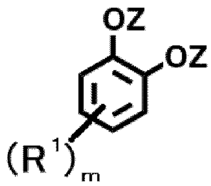

(5)

[Formula 6]

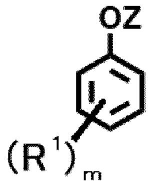

(6)

[Formula 7]

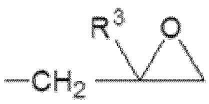

(7)

In the formula (4), Y is represented by a formula (5) or (6), Z is represented by a formula (7), $R^1$ is a hydrogen atom, a hydrocarbon group having 1 to 4 carbon atoms, or an alkoxy group having 1 to 4 carbon atoms, $R^2$ is a hydrogen atom or a methyl group, $R^3$ is a hydrogen atom or a methyl group, m is an integer of 0 to 3, n is an integer of 0 to 4, and p is an integer of 0 to 50.

The epoxy equivalent of the epoxy resin of the present invention is preferably 150 to 300 g/eq.

The present invention relates to a curable resin composition containing the epoxy resin.

The present invention relates to a cured product obtained by a curing reaction of the curable resin composition.

The present invention relates to a fiber-reinforced composite material containing the curable resin composition and reinforcement fibers.

The present invention relates to a fiber-reinforced resin molded article containing the cured product and reinforcement fibers.

Advantageous Effects of Invention

According to the present invention, a cured product obtained using an epoxy resin obtained using a phenolic resin having a specific structure can express low water absorption properties and high bending properties (bending strength, bending elastic modulus, bending strain, etc.), and is useful.

Description of Embodiments

[Phenolic Resin]

The present invention relates to a phenolic resin that is a reaction product of a catechol compound, a phenol compound, and an o-xylylene skeleton-containing compound, and has a catechol skeleton derived from the catechol compound, a phenol skeleton derived from the phenol compound, and an o-xylylene skeleton derived from the o-xylylene skeleton-containing compound.

The phenolic resin preferably includes the catechol skeleton derived from the catechol compound. This is because the phenolic resin is polyfunctional, the cross-linking density is high, and a resultant cured product has heat resistance and high elastic modulus. The phenolic resin preferably includes the phenol skeleton derived from the phenol compound is preferred. This is because the cross-linking density is moderately lower than that of the catechol skeleton, the elongation is high, and therefore the phenolic resin has high strength and low water absorption properties.

The phenolic resin preferably includes the o-xylylene skeleton derived from the o-xylylene skeleton-containing compound. This is because the phenolic resin has a bent structure, an intermolecular space is small, and a cured product obtained using an epoxy resin obtained using the resultant phenolic resin can express increased elastic modulus and high strength (high elongation). In addition, a molecular interaction is moderately low, and the phenolic resin has excellent handlingability at low melt viscosity.
The "phenolic resin" refers to a resin containing a compound having at least a phenolic hydroxy group.
The "catechol skeleton" refers to a skeleton formed by removal from a "skeleton having two hydroxy groups at 1-position and 2-position of an aromatic ring" of a hydrogen atom constituting each of the hydroxy groups.
The "phenol skeleton" refers to a skeleton formed by removal from a "skeleton having a hydroxy group as a substituent in an aromatic ring" of a hydrogen atom constituting the hydroxy group.
The "o-xylylene skeleton" refers to a skeleton having two methylene groups at a positional relationship of ortho position as a substituent in an aromatic ring, the skeleton that connects compounds having at least a phenolic hydroxy group contained in the phenolic resin.

[Catechol Compound]

The phenolic resin is a reaction product of a catechol compound, a phenol compound, and an o-xylylene skeleton-containing compound. The catechol compound is dihydroxybenzene having hydroxy groups at 1-position and 2-position that may have only a hydrogen atom without a substituent on the aromatic ring of the catechol compound, or may have a hydrocarbon group having 1 to 4 carbon atoms or an alkoxy group having 1 to 4 carbon atoms instead of the hydrogen atom as a substituent that may be an alkyl group such as a methyl group or a tert-butyl group. When the catechol compound is used, the phenolic resin includes a catechol skeleton (a catechol skeleton is introduced into the phenolic resin), functional groups are close to each other, and an intermolecular space is small, and therefore, a resultant cured product is estimated to exhibit high elastic modulus. In addition, the cured product becomes polyfunctional, exhibits heat resistance, and thus is useful.
When the substituent is an alkyl group such as a methyl group, the position and number of the substituent are not particularly limited. Since a cured product that is excellent in mechanical strength and low water absorption properties (hydrophobic properties) is obtained, the catechol compound may have a methyl group or a tert-butyl group at 4-position of the catechol. From the viewpoint of bending properties, the catechol compound having a hydrogen atom without a substituent is the most preferred.
The catechol compound may be used alone, or a plurality of catechol compounds in which the positions of alkyl groups such as a methyl group are different may be used in combination.

[Phenol Compound]

The phenolic resin is a reaction product of a catechol compound, a phenol compound, and an o-xylylene skeleton-containing compound. The phenol compound is hydroxybenzene having a hydroxy group that may have only a hydrogen atom without a substituent on the aromatic ring of the phenol compound, or may have a hydrocarbon group having 1 to 4 carbon atoms or an alkoxy group having 1 to 4 carbon atoms instead of the hydrogen atom as a substituent that may be an alkyl group such as a methyl group or a tert-butyl group. When the phenol compound is used, the phenolic resin includes a phenol skeleton (a phenol skeleton is introduced into the phenolic resin), and a resultant cured product exhibits low water absorption properties, and thus is useful.
When the substituent is an alkyl group such as a methyl group, the position and number of the substituent are not particularly limited. Since a cured product that is excellent in low water absorption properties (hydrophobic properties) is obtained, the phenol compound may have a methyl group, a tert-butyl group, or an ethyl group. From the viewpoint of bending properties, the phenol compound having a hydrogen atom without a substituent is the most preferred.
The phenol compound may be used alone, or a plurality of phenol compounds in which the positions of alkyl groups such as a methyl group are different may be used in combination.

[O-Xylylene Skeleton-Containing Compound]

The phenolic resin is a reaction product of a catechol compound, a phenol compound, and an o-xylylene skeleton-containing compound. The phenolic resin preferably includes an o-xylylene skeleton derived from the o-xylylene skeleton-containing compound (the o-xylylene skeleton is introduced into the phenolic resin). This is because a molecular interaction is moderately low, and the phenolic resin having excellent handlingability at low melt viscosity is obtained. In addition, the o-xylylene skeleton is preferably used. This is considered to be because the catechol compound and the phenol compound may be closer to each other, a plurality of functional groups of the resultant phenolic resin are close to each other, and thus a cured product having excellent mechanical strength (increased elastic modulus and high strength

(high elongation)) can be obtained.

When the o-xylylene skeleton-containing compound is used in synthesis of the phenolic resin, a molecular structure is bent in network formation (polymerization) during production of the cured product (during curing) as compared with a case where a p-xylylene skeleton-containing compound or the like is used, and the free volume is small. Therefore, an increase in elastic modulus is presumed. In other words, due to a bent structure, an intermolecular space is small, the molecules are dense, and curing is achieved. Thus, an increase in strength opposed to an increase in elastic modulus can be expressed, both the increase in elastic modulus and the increase in strength can be achieved, and the phenolic resin is useful.

Furthermore, when an effect of decreasing the intermolecular space by incorporation (introduction) of the catechol skeleton and the phenol skeleton is combined with an effect of decreasing the intermolecular space by incorporation (introduction) of the o-xylylene skeleton, the phenolic resin is presumed to exert high bending properties. In particular, the phenolic resin obtained by using the catechol compound and the phenol compound in combination has both a molecular structure having a small space and high elastic modulus and a molecular structure having low cross-linking density and high elongation unlike a case where the catechol compound and the phenol compound are each used alone, and therefore the phenolic resin has high strength and is useful.

Examples of the o-xylylene skeleton-containing compound include o-xylylene dihalide, o-xylylene dialkoxide, o-xylylene glycol, and compounds in which one or more groups on the aromatic rings thereof are substituted by a hydrocarbon group having 1 to 4 carbon atoms. Specific examples thereof include o-xylylene dichloride, o-xylylene dibromide, o-xylylene dimethoxide, o-xylylene diethoxide, and o-xylylene glycol. Among these, from the viewpoint of availability, o-xylylene dichloride and the like are preferred.

The o-xylylene skeleton-containing compound may be used alone, or a plurality of o-xylylene skeleton-containing compounds may be used in combination.

The phenolic resin of the present invention is preferably represented by the following general formula (1).

[Formula 8]

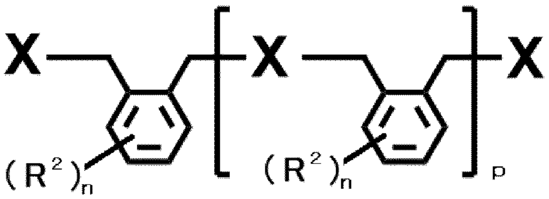

(1)

[Formula 9]

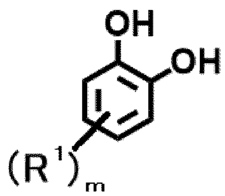

(2)

[Formula 10]

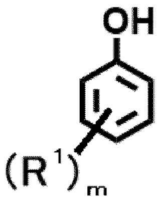

(3)

In the formula (1), X is a catechol compound represented by a formula (2), or a phenol compound represented by a formula (3), $R^1$ is a hydrogen atom, a hydrocarbon group having 1 to 4 carbon atoms, or an alkoxy group having 1 to 4 carbon atoms, $R^2$ is a hydrogen atom or a methyl group, m is an integer of 0 to 3, n is an integer of 0 to 4, and p is an integer of 0 to 50.

As the phenolic resin represented by the general formula (1), for example, the phenolic resin represented by the following general formula (1) can be obtained by a reaction of a catechol compound represented by the following general formula (2), a phenol compound represented by the following general formula (3), and an o-xylylene skeleton-containing compound represented by the following general formula (8) (hereinafter, a halogenated (chlorinated) o-xylylene skeleton-containing compound is exemplified).

[Formula 11]

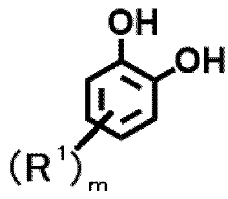

(2)

[Formula 12]

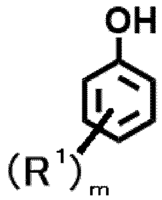

(3)

[Formula 13]

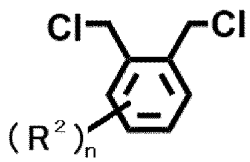

(8)

[Formula 14]

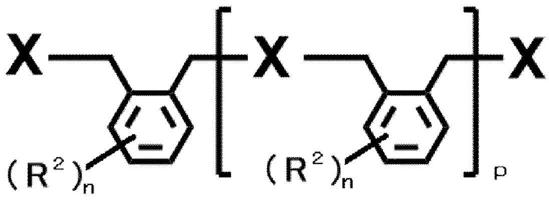

(1)

In the general formula (2) or (3), each $R^1$ as a substituent may be independently a hydrogen atom, a hydrocarbon group having 1 to 4 carbon atoms, or an alkoxy group having 1 to 4 carbon atoms, and from the viewpoint of reactivity in use as a raw material and the bending properties of a cured product, preferred examples of the hydrocarbon group having 1 to 4 carbon atoms include alkyl groups such as a methyl group, an ethyl group, a propyl group, a butyl group, and a t-butyl group. Examples of the alkoxy group having 1 to 4 carbon atoms include a methoxy group, an ethoxy group, a propyloxy group, and a butoxy group. Among these, in a preferred aspect, each $R^1$ is a hydrogen atom since the phenolic resin has low melt viscosity, excellent handlingability, and excellent balance of bending properties and low water absorption properties.

In the general formula (2) or (3), m is an integer of 0 to 3, and preferably an integer of 0 to 2 from the viewpoint of reactivity in use as a raw material and the bending properties of a cured product.

In the general formula (8), each $R^2$ is independently preferably a hydrogen atom or a methyl group, and more preferably a hydrogen atom from the viewpoint of the bending properties and low water absorption properties of a cured product. Each $R^2$ is preferably a hydrogen atom or the like since the bending properties of a cured product are improved. Among these, in a preferred aspect, each $R^2$ is a hydrogen atom since the bending properties are improved.

In the general formula (8), n is an integer of 0 to 4, and preferably an integer of 0 to 1 from the viewpoint of reactivity in use as a raw material and the bending properties of a cured product.

In the general formula (1), p is an integer of 0 to 50, and preferably an integer of 0 to 20 from the viewpoint of improvement of the bending properties of a cured product. When p is 0, the phenolic resin contains a compound having one catechol skeleton and one phenol skeleton.

Details of $R^1$, $R^2$, m, and n in the general formula (1) are the same as those in the general formulae (2) or (3), and (8).

As the reaction ratio of the o-xylylene skeleton-containing compound to the catechol compound and the phenol compound, the total amount of the catechol compound and the phenol compound is preferably within the range of 2 to 50 mol, and more preferably 4 to 20 mol, relative to 1 mol of the o-xylylene skeleton-containing compound since an epoxy resin that is excellent in a balance of melt viscosity, and the bending properties, heat resistance, and low water absorption properties of a cured product is obtained.

A reaction of the catechol compound and the phenol compound with the o-xylylene skeleton-containing compound is performed preferably in the presence of an acid catalyst since the reaction efficiently advances. However, when as the o-xylylene skeleton-containing compound, o-xylylene dichloride or the like is used, the reaction sufficiently advances without an acid catalyst. Therefore, in such a case, a catalyst may not be used. Examples of the acid catalyst include inorganic acids such as hydrochloric acid, sulfuric acid, and phosphoric acid, organic acids such as methanesulfonic acid, p-toluenesulfonic acid, and oxalic acid, and Lewis acids such as boron trifluoride, aluminum chloride anhydride, and zinc chloride. In this case, the amount of used acid catalyst is preferably within the range of 0.01 to 5% by mass relative to the total mass of reaction raw materials.

The reaction of the catechol compound and the phenol compound with the o-xylylene skeleton-containing compound (here when a chlorinated o-xylylene skeleton-containing compound is used) is generally performed under a temperature condition of 50 to 180°C. Desirably, a hydrogen chloride gas generated at that time is rapidly released to the outside of the system and neutralized by alkali water or the like, resulting in detoxification. A reaction time is a time required until the generation of hydrogen chloride gas substantially ends, the o-xylylene skeleton-containing compound as the raw material is consumed, and a chlorine component derived from the o-xylylene skeleton-containing compound is not detected. The reaction time depends on the reaction temperature, but is generally about 1 to 50 hours. The reaction may be performed in an organic solvent, if necessary. The organic solvent used in this case is not particularly limited as long as it can be used under the temperature condition. Specific examples thereof include methanol, ethanol, isopropanol, n-butanol, methyl cellosolve, ethyl cellosolve, butyl cellosolve, 1-methoxy-2-propanol, diglyme, diethylene glycol, diethylene glycol monomethyl ether, diethylene glycol monoethyl ether, toluene, xylene, and methyl isobutyl ketone. When the organic solvent is used, the amount of the organic solvent used is preferably within the range of 5 to 500% by mass relative to the total mass of reaction raw materials.

After completion of the reaction of the catechol compound and the phenol compound with the o-xylylene skeleton-containing compound, unreacted reaction materials and solvents, and the like are removed, to obtain the phenolic resin.

When part of the phenol compound remains in an unreacted state after completion of the reaction of the catechol compound and the phenol compound with the o-xylylene skeleton-containing compound, voids and the like may be generated by volatilization of the residual catechol compound and the residual phenol compound during production of a cured product. Therefore, it is not preferable that the residual amounts of the catechol compound and the phenol compound be increased. Accordingly, the total content ratio in GPC area% of the residual (unreacted) catechol compound and the residual (unreacted) phenol compound that is calculated by GPC measurement is preferably 1 area% or less, more preferably 0.8 area% or less, and even more preferably 0.4 area% or less. The GPC area% as used herein refers to a value obtained by dividing the GPC peak area value of the residual (unreacted) catechol compound or the residual (unreacted) phenol compound by the total GPC peak area value of all the components.

The hydroxy group equivalent of the phenolic resin is preferably 90 to 140 g/eq, more preferably 92 to 138 g/eq, and even more preferably 94 to 136 g/eq. The hydroxy group equivalent is preferably within the aforementioned range since a resultant cured product has excellent balance of heat resistance, moisture resistance (water absorption ratio and bending properties after water absorption), and mechanical strength. The hydroxy group equivalent of the phenolic resin is based on a method for measuring the "hydroxy group equivalent of a phenolic resin" in Examples below.

The melt viscosity (150°C) of the phenolic resin is preferably 4.0 dPa·s or less, more preferably 3.0 dPa·s or less, and even more preferably 0.1 to 2.0 dPa·s. The melt viscosity of the phenolic resin falling within the aforementioned range is preferred. This is because the phenolic resin is excellent in flowability and handlingability at low viscosity, and therefore an epoxy resin synthesized from the phenolic resin as the raw material has low viscosity, and is excellent in handlingability

during production of a cured product. The melt viscosity (150°C) as used herein is measured with an ICI viscometer in accordance with ASTM D4287.

The softening point of the phenolic resin is preferably 50 to 100°C, and more preferably 50 to 80°C. The softening point of the phenolic resin falling within the aforementioned range is preferred since the phenolic resin is excellent in handlingability and storage stability. The softening point as used herein is measured in accordance with JIS K7234 (ring and ball method).

<Epoxy Resin>

An epoxy resin of the present invention is preferably an epoxy resin that is a reaction product that has a glycidyl ether group obtained by a reaction of a phenolic hydroxy group of the phenolic resin with epihalohydrin, and is represented by the following general formula (4). The "phenolic hydroxy group" as used herein refers to a hydroxy group contained in the catechol skeleton and the phenol skeleton.

[Formula 15]

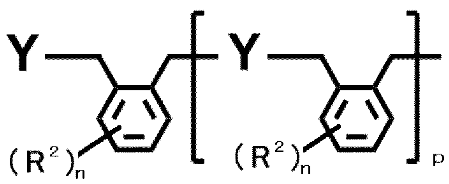

(4)

[Formula 16]

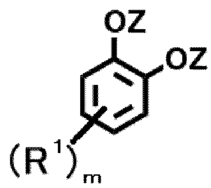

(5)

[Formula 17]

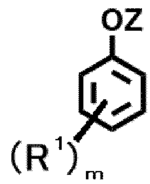

(6)

[Formula 18]

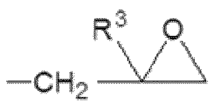

(7)

In the general formula (4), Y is represented by the general formula (5) or (6), Z is represented by the general formula (7), $R^1$ is a hydrogen atom, a hydrocarbon group having 1 to 4 carbon atoms, or an alkoxy group having 1 to 4 carbon atoms, $R^2$ is a hydrogen atom or a methyl group, $R^3$ is a hydrogen atom or a methyl group, m is an integer of 0 to 3, n is an integer of 0 to 4, and p is an integer of 0 to 50.

Details of $R^1$, $R^2$, m, n, and p in the general formulae (4) to (6) are the same as those in the general formulae (1) to (3).

In the general formulae (5) and (6), Z is represented by the general formula (7), and in the general formula (7), each $R^3$ is independently preferably a hydrogen atom or a methyl group, and more preferably a hydrogen atom. When each

$R^3$ is a hydrogen atom or the like, a curing reaction with a curing agent advances smoothly, and the epoxy resin is useful.

The epoxy resin is an epoxy resin having a glycidyl ether group introduced by a reaction of the phenolic hydroxy group of the phenolic resin with epihalohydrin, and a cured product obtained using the epoxy resin is excellent in heat resistance, low water absorption properties, and high bending properties (bending strength, bending elastic modulus, bending strain, etc.), and is preferred.

The "epoxy resin" refers to a resin containing a compound having at least the glycidyl ether group.

By a reaction of the phenolic resin with the epihalohydrin, a targeted epoxy resin can be obtained. The reaction is performed, for example, by a method in which the epihalohydrin is used at the proportion of 2 to 10 mol relative to 1 equivalent of the phenolic hydroxy group of the phenolic resin, and the reaction is performed at a temperature of 20 to 120°C for 0.5 to 12 hours while a basic catalyst in an amount of 0.9 to 2.0 mol relative to 1 equivalent of the phenolic hydroxy group is added at once or dividedly.

In industrial production, the whole amount of the epihalohydrin used for preparation in a first batch of production of the epoxy resin is new, and after the next batch, epihalohydrin collected from a crude reaction product and a new epihalohydrin in an amount corresponding to the amount of the epihalohydrin consumed in the reaction are preferably used in combination. At that time, the used epihalohydrin is not particularly limited. Examples thereof include epichlorohydrin, epibromohydrin, and β-methylepichlorohydrin. Among these, epichlorohydrin is preferred since it is industrially easily available.

Specific examples of the basic catalyst include an alkaline earth metal hydroxide, an alkali metal carbonate, and an alkali metal hydroxide. Among these, an alkali metal hydroxide is preferred from the viewpoint of excellent catalytic activity, and specifically, sodium hydroxide and potassium hydroxide are preferred.

After completion of the reaction, the reaction mixture is washed with water, and an unreacted epihalohydrin and the organic solvent are then removed by distillation under heating and reduced pressure. In order to obtain the epoxy resin in which the amount of hydrolyzable halogen is considerably small, the obtained epoxy resin is dissolved again in the organic solvent, and an aqueous solution of an alkali metal hydroxide such as sodium hydroxide or potassium hydroxide is added resulting in an additional reaction. At that time, a phase-transfer catalyst such as a quaternary ammonium salt or a crown-ether may be used in order to increase the reaction rate. When the phase-transfer catalyst is used, the amount of the phase-transfer catalyst used is preferably 0.1 to 3.0 parts by mass relative to 100 parts by mass of the epoxy resin. After completion of the reaction, a produced salt is removed by filtration, waterwashing, and the like, and the organic solvent is removed under heating and reduced pressure, to obtain the intended epoxy resin of the present invention.

The epoxy equivalent of the epoxy resin is preferably 150 to 300 g/eq, more preferably 160 to 280 g/eq, and even more preferably 180 to 250 g/eq. The epoxy equivalent of the epoxy resin falling within the aforementioned range is preferred. This is because the cross-linking density of a cured product is moderate, the resultant cured product is excellent in a balance of heat resistance, low water absorption properties, and bending properties. The epoxy equivalent as used herein is measured in accordance with JIS K7236.

The melt viscosity (150°C) of the epoxy resin is preferably 2.0 dPa·s or less, more preferably 1.5 dPa·s or less, and even more preferably 1.0 dPa·s or less. The melt viscosity of the epoxy resin falling within the aforementioned range is preferred since the epoxy resin is excellent in flowability and handlingability at low viscosity, and a resultant cured product is also excellent in moldability. The melt viscosity (150°C) as used herein is measured with an ICI viscometer in accordance with ASTM D4287.

<Curable Resin Composition>

The present invention relates to a curable resin composition containing the epoxy resin. Since the curable resin composition contains the epoxy resin, the curable resin composition is excellent in handlingability at low viscosity, and a resultant cured product has heat resistance, low water absorption properties, and high bending properties (bending strength, bending elastic modulus, etc.), and is useful.

In addition to the epoxy resin, the curable resin composition of the present invention may contain an additional resin (containing other epoxy resins), a curing agent, an additive, a solvent, and the like as long as the effects of the present invention are not impaired.

<Other Epoxy Resins>

As the other epoxy resins, various epoxy resins can be used. Examples thereof include naphthalene skeleton-containing epoxy resins such as 2,7-diglycidyloxynaphthalene, an α-naphthol novolac type epoxy resin, a β-naphthol novolac type epoxy resin, an α-naphthol/β-naphthol co-condensed novolac polyglycidyl ether, a naphthol aralkyl type epoxy resin, and 1,1-bis(2,7-diglycidyloxy-1-naphthyl)alkane; bisphenol type epoxy resins such as a bisphenol A type epoxy resin, and a bisphenol F type epoxy resin; biphenyl type epoxy resins such as a biphenyl type epoxy resin, and a tetramethyl biphenyl type epoxy resin; novolac type epoxy resins such as a phenol novolac type epoxy resin, a cresol

novolac type epoxy resin, a bisphenol A novolac type epoxy resin, and a biphenyl novolac type epoxy resin; tetraphenylethane type epoxy resins; dicyclopentadiene-phenol-addition reaction type epoxy resins; phenol aralkyl type epoxy resins; and phosphorus atom-containing epoxy resins. Examples of the phosphorus atom-containing epoxy resins include an epoxidized product of 9,10-dihydro-9-oxa-10-phosphaphenanthrene-10-oxide (hereinafter abbreviated as "HCA"), an epoxidized product of a phenolic resin obtained by a reaction of HCA and quinones, an epoxy resin obtained by modifying a phenol novolac type epoxy resin with HCA, an epoxy resin obtained by modifying a cresol novolac type epoxy resin with HCA, and an epoxy resin obtained by modifying a bisphenol A type epoxy resin with a phenolic resin obtained by a reaction of HCA and quinones. The other epoxy resins may be each used alone, or two or more types thereof may be used in combination.

[Additional Resin]

In addition to the epoxy resin and the other epoxy resins, the curable resin composition of the present invention may contain an additional resin. The "additional resin" herein means a resin other than the epoxy resin.
Specific examples of the additional resin include, but not particularly limited to, an active ester, a maleimide resin, a bismaleimide resin, a polymaleimide resin, a polyphenylene ether resin, a polyimide resin, a cyanate ester resin, a benzoxazine resin, a triazine-containing cresol novolac resin, a cyanate ester resin, a styrenemaleic anhydride resin, an allyl group-containing resin such as diallylbisphenol or triallylisocyanurate, a polyphosphate ester, and a phosphate-carbonate copolymer. The additional resins may be used alone, or two or more types thereof may be used in combination.

[Curing Agent]

The epoxy resin composition of the present invention may contain a curing agent with the epoxy resin. When the curing agent is contained, a cured product having excellent heat resistance, low water absorption properties, and bending properties can be obtained.
Examples of the curing agent used herein include an amine compound, an amide compound, an acid anhydride, and a phenolic resin. The curing agents may be each used alone, or two or more types thereof may be used in combination. As a phenolic resin that is the curing agent, the phenolic resin represented by the general formula (1) can also be used.
Examples of the amine compound include diaminodiphenylmethane, diethylenetriamine, triethylenetetramine, diaminodiphenyl sulfone, isophoronediamine, imidazole, a $BF_3$-amine complex, and a guanidine derivative.
Examples of the amide-based compound include dicyandiamide, and a polyamide resin synthesized from carboxylic acid compound such as an aliphatic dibasic acid, a dimer acid, or a fatty acid with an amine such as ethylenediamine.
Examples of the acid anhydrides include phthalic anhydride, trimellitic anhydride, pyromellitic anhydride, maleic anhydride, tetrahydrophthalic anhydride, methyltetrahydrophthalic anhydride, methyl nadic anhydride, hexahydrophthalic anhydride, and methylhexahydrophthalic anhydride.
Examples of the phenolic resin include polyhydric phenol compounds such as a phenol novolac resin, a cresol novolac resin, an aromatic hydrocarbon formaldehyde resinmodified phenolic resin, a dicyclopentadiene phenol-added resin, a phenol aralkyl resin (xylok resin), a polyhydric phenolic novolac resin that is typified by a resorcin novolac resin, and synthesized from a polyhydric hydroxy compound and formaldehyde, a naphthol aralkyl resin, a trimethylolmethane resin, a tetraphenylolethane resin, a naphthol novolac resin, a naphthol-phenol co-condensed novolac resin, a naphthol-cresol co-condensed novolac resin, a biphenyl-modified phenolic resin (a polyhydric phenol compound having a phenolic nucleus linked with a biphenyl skeleton through a bismethylene group), a biphenyl-modified naphthol resin (a polyhydric naphthol compound having a phenolic nucleus linked with a biphenyl skeleton through a bismethylene group), an aminotriazine-modified phenolic resin (a polyhydric phenol compound having a phenolic nucleus linked through melamine, benzoguanamine, or the like), and an alkoxy group-containing aromatic ring-modified novolac resin (a polyhydric phenol compound in which a phenolic nucleus and an alkoxy group-containing aromatic ring are linked through formaldehyde).
The curing agents may be each used alone, or two or more types thereof may be used in combination.
As the mixing ratio of the epoxy resin component and the curing agent in the curable resin composition of the present invention, the amount of an active group of the curing agent relative to 1 equivalent (epoxy equivalent) in total of the epoxy group of the epoxy resin component is preferably 0.7 to 1.5 eq since a cured product having excellent curing properties, heat resistance, low water absorption properties, and toughness is obtained.
Examples of the additive include various additives such as a curing accelerator, a flame retardant, an inorganic filler, a silane coupling agent, a release agent, a pigment, an emulsifier, and a solvent. If necessary, the additive can be contained in the curable resin composition.

[Curing Accelerator]

Examples of the curing accelerator include a phosphorus compound, a tertiary amine, imidazole, an organic acid metal salt, a Lewis acid, and an amine complex salt. Among these, from the viewpoint of excellent curing properties, heat resistance, low water absorption properties, electrical characteristics, moisture-resistant reliability, and the like, 2-ethyl-4-methylimidazole that is the imidazole compound, triphenylphosphine that is the phosphorus compound, and 1,8-diazabicyclo-[5.4.0]-undecene (DBU) that is the tertiary amine are preferred.

[Flame Retardant]

Examples of the flame retardant include inorganic phosphorus compounds such as red phosphorus, ammonium phosphate such as monoammonium polyphosphate, diammonium polyphosphate, triammonium polyphosphate, and ammonium polyphosphate, and phosphoric acid amide; organic phosphorus compounds such as a phosphate ester compound, a phosphonic acid compound, a phosphinic acid compound, a phosphine oxide compound, a phosphorane compound, and an organic nitrogen-containing phosphorus compound, cyclic organic phosphorus compounds such as 9,10-dihydro-9-oxa-10-phosphaphenanthrene-10-oxide, 10-(2,5-dihydroxyphenyl)-10H-9-oxa-10-phosphaphenanthrene-10-oxide, and 10-(2,7-dihydroxynaphthyl)-10H-9-oxa-10-phosphaphenanthrene-10-oxide, and derivatives obtained by a reaction of the compounds with a compound such as an epoxy resin or a phenolic resin; nitrogen-containing flame retardants such as a triazine compound, a cyanuric acid compound, an isocyanuric acid compound, and phenothiazine; silicone-based flame retardants such as a silicone oil, a silicone rubber, and a silicone resin; and inorganic flame retardants such as a metal hydroxide, a metal oxide, a metal carbonate compound, a metal powder, a boron compound, and a lowmelting-point glass. When the flame retardant is used, the amount thereof in the curable resin composition is preferably within the range of 0.1 to 20% by mass.

[Inorganic Filler]

The inorganic filler is mixed, for example, when the curable resin composition of the present invention is used for a semiconductor sealing material. Examples of the inorganic filler include molten silica, crystalline silica, alumina, silicon nitride, and aluminum hydroxide. Among these, the molten silica is preferred since a larger amount of inorganic filler can be mixed. Although any of crushed or spherical molten silica can be used, it is preferable that the spherical molten silica be mainly used to increase the amount of the molten silica mixed and to suppress an increase in the melt viscosity of the curable resin composition. Furthermore, it is preferable that the particle size distribution of the spherical silica be properly adjusted to increase the amount of the spherical silica mixed. The filling rate of the inorganic filler mixed in 100 parts by mass of the curable resin composition is preferably within the range of 0.5 to 95 parts by mass.

The curable resin composition of the present invention may be prepared without a solvent or may contain a solvent. The solvent has a function of adjusting the viscosity of the curable resin composition, and the like. Specific examples of the solvent include, but not particularly limited to, ketone-based solvents such as acetone, methyl ethyl ketone, methyl isobutyl ketone, and cyclohexanone; ether-based solvents such as diethyl ether and tetrahydrofuran; esterbased solvents such as ethyl acetate, butyl acetate, cellosolve acetate, propylene glycol monomethyl ether acetate, and carbitol acetate; carbitols such as cellosolve and butyl carbitol, aromatic hydrocarbons such as toluene, xylene, ethylbenzene, mesitylene, 1,2,3-trimethylbenzene, and 1,2,4-trimethylbenzene, and amide-based solvents such as dimethylformamide, dimethylacetamide, and N-methylpyrrolidone. The solvents may be used alone, or two or more types thereof may be used in combination.

When the curable resin composition of the present invention is used for a conductive paste and the like, a conductive filler such as silver powder or copper powder can be used.

<Cured Product>

The present invention relates to a cured product obtained by a curing reaction of the curable resin composition. The cured product is preferred. This is because the cured product can be obtained from the curable resin composition containing the epoxy resin, and the cured product can exhibit heat resistance, low water absorption properties, and high bending elastic modulus (bending strength, bending elastic modulus, bending strain, etc.).

The curable resin composition is obtained by uniformly mixing the respective components described above. The cured product can be easily obtained by the same method as a conventionally known method in which the epoxy resin component, the curing agent, and if necessary, the curing accelerator, and the like, are mixed and the mixture is used. Examples of the resultant cured product include molded cured products such as a laminate, a casting product, an adhesive layer, a coated film, and a film.

In a method for preparing the cured product by a curing reaction of the curable resin composition, for example, a

heating temperature during heating and curing is not particularly limited, and is preferably 100 to 300°C, and a heating time is preferably 1 to 24 hours.

<Application of Curable Resin Composition>

Examples of applications for which the curable resin composition is used include a printed wiring board material, a resin composition for a flexible wiring substrate, an interlayer insulating material for a build-up substrate, an insulating material for a circuit board such as an adhesive film for build-up, a resin casting material, an adhesive, a semiconductor sealing material, a semiconductor device, a prepreg, a conductive paste, a build-up film, a build-up substrate, a fiber-reinforced composite material, and a molded article (fiber-reinforced resin molded article) obtained by curing the composite material. For a printed wiring board material, an insulating material for a circuit board, and an adhesive film for build-up among the applications, the curable resin composition can be used as an insulating material for what is called a substrate for built-in electronic parts in which a passive part such as a capacitor, or an active part such as an IC chip is embedded in a substrate. Among these, by utilizing properties such as excellent heat resistance, low water absorption properties, and high elastic modulus of the cured product, the curable resin composition of the present invention is preferably used for a semiconductor sealing material, a semiconductor device, a prepreg, a flexible wiring substrate, a circuit board, and a build-up film, a build-up substrate, a multilayered printed wiring board, a fiber-reinforced composite material, and a molded article obtained by curing the composite material. Hereinafter, a method for producing the fiber-reinforced composite material and the like from the curable resin composition will be described.

1. Semiconductor Sealing Material

Examples of a method for preparing a semiconductor sealing material from the curable resin composition include a method in which the curable resin composition, and a compounding agent such as the curing accelerator and the inorganic filler are sufficiently melt-mixed using an extruder, a kneader, a roll, or the like, if necessary, until the components are homogeneous. In this case, as the inorganic filler, molten silica is generally used. When the molten silica is used as a high thermal conductive semiconductor sealing material for a power transistor or a power IC, filling of crystalline silica, alumina, or silicon nitride having higher thermal conductivity than molten silica may be increased, or molten silica, crystalline silica, alumina, silicon nitride, or the like may be used. The inorganic filler is used preferably at a filling rate of 30 to 95 parts by mass, and in terms of improving flame retardancy, moisture resistance, and resistance to solder cracking, and decreasing a linear expansion coefficient, more preferably 70 parts by mass or more, and even more preferably 80 parts by mass or more, relative to 100 parts by mass of the curable resin composition.

2. Semiconductor Device

Examples of a method for preparing a semiconductor device from the curable resin composition include a method in which the semiconductor sealing material is casted or molded with a transfer molding machine, an injection molding machine, or the like and then heated at 50 to 200°C for 2 to 10 hours.

3. Prepreg

Examples of a method for preparing a prepreg from the curable resin composition include a method in which with a varnish of the curable resin composition that is obtained by mixing the following organic solvent, a reinforcing base material (paper, glass cloth, glass nonwoven fabric, aramid paper, aramid cloth, glass mat, glass roving cloth, etc.) is impregnated, and then heated at heating temperature depending on the type of the used solvent, preferably 50 to 170°C, to prepare the prepreg. The mass ratio of the resin composition and the reinforcing base material used at that time is not particularly limited. It is preferable that the prepreg be prepared such that the ratio of the resin in the prepreg is generally 20 to 60% by mass.

Examples of the organic solvent used herein include methyl ethyl ketone acetone, dimethylformamide, methyl isobutyl ketone, methoxy propanol, cyclohexanone, methyl cellosolve, ethyl diglycol acetate, and propylene glycol monomethyl ether acetate. The organic solvent and an appropriate use amount can be appropriately selected depending on applications. For example, when a printed circuit board is produced from the prepreg as described below, a polar solvent having a boiling point of 160°C or lower, such as methyl ethyl ketone, acetone, or dimethylformamide is preferably used, and the polar solvent is preferably used at such a ratio that the nonvolatile content is 40 to 80% by mass.

4. Circuit Board

Examples of a method for preparing a printed circuit board from the curable resin composition include a method in

which the prepreg is layered by an ordinary method, copper foil is appropriately overlaid, and they are thermally compression-bonded application of a pressure of 1 to 10 MPa at 170 to 300°C for 10 minutes to 3 hours.

5. Flexible Wiring Substrate

Examples of a method for producing a flexible wiring substrate from the curable resin composition include a production method including three steps described below. A first step is a step of applying a curable resin composition containing an active ester, an epoxy resin, and an organic solvent to an electrically insulating film using a coater such as a reverse roll coater, or a comma coater; a second step is a step of heating the electrically insulating film having the applied curable resin composition at 60 to 170°C for 1 to 15 minutes using a heater and volatilizing the solvent from the electrically insulating film, to obtain the curable resin composition in a B-stage state; and a third step is a step of thermocompression-bonding the electrically insulating film having the curable resin composition in the B-stage state with metal foil through an adhesive using a heating roll or the like (a thermocompression-bonding pressure is preferably 2 to 200 N/cm, and a thermocompression-bonding temperature is preferably 40 to 200°C). If sufficient adhesion performance is achieved through the three steps, the operation may be completed. If complete adhesion performance is required, post-curing under conditions of 100 to 200°C and 1 to 24 hours is preferably further performed. The thickness of a finally cured film of the curable resin composition is preferably within the range of 5 to 100 μm.

6. Build-Up Substrate

Examples of a method for producing a build-up substrate from the curable resin composition include a production method including three steps described below. A first step is a step of applying the curable resin composition appropriately containing a rubber, a filler, or the like to a circuit board having a circuit by a method such as a spray coating method or a curtain coating method, followed by curing; a second step is a step of drilling the circuit board to form a predetermined through hole section or the like, if necessary, then treating the circuit board with a roughening agent, washing the surface of the circuit board with hot water, to form irregularities, and plating the circuit board with a metal such as copper; and a third step is a step of sequentially repeating the operation, if desired, and alternately building up a resin insulating layer and a conductor layer with a certain circuit pattern to form the build-up substrate. It is preferable that drilling for the through hole section be performed after formation of an outermost resin insulating layer. In addition to application of the solution as described above, the first step can be performed by a method for laminating a build-up film having a desired thickness that is formed by applying and drying in advance. The build-up substrate of the present invention can also be produced by thermally compression-bonding a copper foil with a resin that is obtained by semi-curing the resin composition on the copper foil on a wiring substrate having a circuit at 170 to 250°C without forming a roughened surface and a plating treatment.

7. Build-Up Film

Examples of a method for producing a build-up film from the curable resin composition include a method in which the curable resin composition is applied to a support film, to form a curable resin composition layer as an adhesive film for a multilayered printed wiring board.

When a build-up film is produced from the curable resin composition, it is important that the film is softened under a temperature condition of lamination in a vacuum laminating method (generally at 70 to 140°C), and the film exhibits flowability (resin flow) capable of filling a via hole or a through hole in a circuit board with a resin, contemporaneously with the lamination of the circuit board. For expression of such properties, the aforementioned components are preferably mixed.

It is preferable that a through hole that generally has a diameter of 0.1 to 0.5 mm and a depth of 0.1 to 1.2 mm of a multilayered printed wiring board be filled with the resin so as to generally fall within this range. When both surfaces of the circuit board are subjected to lamination, about a half of the through hole is desirably filled.

In the method for producing the adhesive film, specifically, a varnish of the curable resin composition is prepared, and then applied to a surface of a support film (Y), and an organic solvent is dried by heating, blowing hot air, or the like, to form a composition layer (X) of the curable resin composition. Thus, the adhesive film can be produced.

In general, the thickness of the formed composition layer (X) is preferably equal to or more than the thickness of the conductor layer. Since the thickness of the conductor layer in the circuit board is generally within the range of 5 to 70 μm, the thickness of the resin composition layer is preferably within the range of 10 to 100 μm.

The composition layer (X) in the present invention may be protected by a protective film described below. Protection by the protective film can prevent a surface of the resin composition layer from suffering attachment of contaminants or the like or from being scratched.

Examples of the support film and the protective film include polyolefines such as a polyethylene, a polypropylene,

and a polyvinyl chloride, polyesters such as a polyethylene terephthalate (hereinafter also referred to as "PET") and a polyethylene naphthalate, polycarbonates, polyimides, a release paper, and metal foils such as a copper foil and an aluminum foil. The support film and the protective film may be subjected to a mad treatment and a corona treatment as well as a releasing treatment.

The thickness of the support film is not particularly limited, and the support film generally having a thickness of the range of 10 to 150 um, and preferably 25 to 50 um is used. The thickness of the protective film is preferably 1 to 40 μm.

The support film (Y) is peeled after lamination on the circuit board or formation of an insulating layer by heating and curing. When the adhesive film is heated and cured and the support film (Y) is then peeled, attachment of contaminants or the like at a curing step can be prevented. When the support film is peeled after curing, the support film is generally subjected to a releasing treatment in advance.

8. Multilayered Printed Wiring Board

A multilayered printed wiring board can also be produced by using the film obtained as described above. In a method for producing such a multilayered printed wiring board, for example, if the composition layer (X) is protected by the protective film, the composition layer (X) and the protective film are peeled, the composition layer (X) is laminated directly on a surface or both surfaces of the circuit board, for example, by a vacuum lamination method. The lamination method may be in a batch mode or a continuous mode with rolls. Before lamination, the adhesive film and the circuit board may be heated (preheated), if necessary.

The lamination is preferably performed under a lamination condition of a compression-bonding temperature (lamination temperature) of 70 to 140°C, a compression-bonding pressure of 1 to 11 $kgf/cm^2$ ($9.8\times10^4$ to $107.9\times10^4$ $N/m^2$), and a reduced pressure of air pressure of 20 mm Hg (26.7 hPa) or less.

9. Fiber-Reinforced Composite Material

The present invention relates to a fiber-reinforced composite material containing the curable resin composition and reinforcement fibers. In a method for producing a fiber-reinforced composite material from the curable resin composition, the components constituting the curable resin composition are uniformly mixed to prepare a varnish, a reinforced base material formed from reinforcement fibers is then impregnated with the varnish, and a polymerization reaction is performed to produce a fiber-reinforced composite material. Since the epoxy resin of the present invention is especially excellent in handlingability due to low melt viscosity, the epoxy resin is suitable for production of the fiber-reinforced composite material, and is preferred.

Specifically, a curing temperature in such a polymerization reaction is preferably within the range of 50 to 250°C. In particular, it is preferable that at 50 to 100°C, the curable resin composition be cured, to obtain a tack-free cured product, and the cured product be treated under a temperature condition of 120 to 200°C.

The reinforcement fibers as used herein may be any of a twisted yarn, an untwisted yarn, or a non-twisted yarn. The untwisted yarn and the non-twisted yarn are preferred since they have both moldability and mechanical strength of a fiber-reinforced plastic member. As a form of the reinforcement fibers, fibers in which fiber directions are aligned in one direction, or a fabric can be used. The fabric can be freely selected from a plain weave, a sateen weave, and the like according to a place to be used and an application. Specific examples of the reinforcement fibers include carbon fibers, glass fibers, aramid fibers, boron fibers, alumina fibers, and silicon carbide fibers, which have excellent mechanical strength and durability. Two or more of these fibers can be used in combination. Among these, carbon fibers are preferred since a molded article has favorable strength. As the carbon fibers, any fibers such as polyacrylonitrile-based, pitch-based, or rayon-based fibers can be used. Among these, polyacrylonitrile-based fibers that easily form highly-strong carbon fibers are preferred. When the reinforced base material formed from the reinforcement fibers is impregnated with the varnish to from the fiber-reinforced composite material, the amount of the reinforcement fibers used is preferably such an amount that the volume content of the reinforcement fibers in the fiber-reinforced composite material is within the range of 40 to 85%.

10. Fiber-Reinforced Resin Molded Article

The present invention relates to a fiber-reinforced resin molded article including the cured product and the reinforcement fibers. Examples of a method for producing a fiber-reinforced resin molded article from the curable resin composition include a method in which a prepreg in which the reinforcement fibers is impregnated with the varnish is produced by a hand layup method and a spraying up method in which a fibrous aggregate is laid on a mold, and the varnish is multilayered, a vacuum bagging method in which while substrates made of reinforcement fibers are layered with the substrate being impregnated with the varnish using a convex or concave mold, molded, covered with a flexible mold capable of applying a pressure to a molded object, airtightly sealed, and molded in vacuum (under reduced pressure), a SMC pressing method in which the varnish in which reinforcement fibers are added in advance is molded into a sheet, and

compression-molded with a die, a RTM method in which the varnish is poured in a combined mold formed by placing fibers, or the like, and the prepreg is baked and hardened in a large autoclave. Since the epoxy resin of the present invention is excellent in handlingability due to low melt viscosity, the epoxy resin is especially suitable for production of the fiber-reinforced resin molded article, and is preferred. The fiber-reinforced resin molded article obtained as described above is a molded article including reinforcement fibers and the cured product of the curable resin composition. Specifically, the amount of the reinforcement fibers in the fiber-reinforced resin molded article is preferably within the range of 40 to 70% by mass, and in terms of strength, particularly preferably within the range of 50 to 70% by mass.

11. Others

The methods for producing the semiconductor sealing material, the fiber-reinforced composite material, and the like are described above, but other cured products can also be produced from the curable resin composition. The other cured products can be produced in accordance with a general method for curing the curable resin composition that is a method for producing the other cured products. For example, a heating temperature condition may be appropriately selected depending on the type, application, and the like of combined curing agent.

## Examples

Hereinafter, the present invention will be described more specifically by Examples and Comparative Examples, but the present invention is not intended to be limited to Examples and Comparative Examples. In the following description, amounts are based on mass unless otherwise specified. A phenolic resin and an epoxy resin obtained as described below, and a cured product obtained using the epoxy resin were subjected to measurement and evaluation under the following conditions and the like.

<Hydroxy Group Equivalent of Phenolic Resin>

The hydroxy group equivalent (g/eq) of a phenolic resin was determined by the following procedure and equation.
In a 500-mL Erlenmeyer flask, 2.5 g of a phenolic resin obtained as described below as a sample, 7.5 g of triphenylphosphine, 7.5 g of pyridine, and 2.5 g of acetic anhydride were precisely weighted, and treated at 120°C for 2.5 hours, and 5 mL of distilled water was then added to terminate a reaction. Subsequently, the resultant was dissolved in 150 mL of tetrahydrofuran and 200 mL of propylene glycol monomethyl ether, to prepare a sample solution.
Separately from the sample solution, 10 mL of distilled water and 2.5 g of acetic anhydride were precisely weighted, 10 g of pyridine was added, the mixture was stirred for 10 minutes and allowed to stand for 20 minutes, and 150 mL of tetrahydrofuran and 200 mL propylene glycol monomethyl ether were mixed in the mixture, to prepare a blank solution.
For the obtained sample solution and the blank solution, titration was performed using a 0.5 mol/L potassium hydroxide ethanolic solution (titrant) with an automatic potentiometric titrator AT-510 (manufactured by Kyoto Electronics Manufacturing Co., Ltd.), and the hydroxy group equivalent was calculated by the following equation.

BL = (titer of blank solution) × (theoretical titer of 2.5 g of acetic anhydride)/(theoretical titer of blank solution) = Y × [(2.5/102.09) × 2 × (1000/5)]/[(W/102.09) × 2 × (1000/5)] [Blank Equation]

BL: blank value (mL)
Y: titer (mL) of blank solution
W: amount (g) of acetic anhydride in blank measurement

[Equation for Calculation of Hydroxy Group Equivalent of Sample]

Hydroxy group equivalent (g/eq) = S × 1000/[{(BL×FA3/FA5) - EP1} × FA4]

S: amount (g) of sample

FA3: amount (g) of acetic anhydride in sample measurement
FA4: concentration (mol/L) of titrant (KOH ethanolic solution)
FA5: amount (g) of acetic anhydride in sample measurement
EP1: titer (mL) of sample

<Softening Point of Phenolic Resin>

A softening point (°C) was measured in accordance with JIS K 7234 (ring and ball method).

<Measurement of Melt Viscosity (150°C)>

A melt viscosity (dPa·s) at 150°C was measured with an ICI viscometer in accordance with ASTM D4287.

<Measurement of Viscosity (25°C)>

A viscosity (mPa·s) at 25°C was measured with an E-type viscometer (TV-22 manufactured by TOKI SANGYO CO., LTD.).

<Epoxy Equivalent of Epoxy Resin>

The epoxy equivalent (g/eq) of an epoxy resin obtained as described below was measured in accordance with JIS K 7236.

<Measurement of GPC>

A phenolic resin obtained by a synthesis method described below, and an epoxy resin obtained using the phenolic resin were subjected to GPC measurement with the following measurement apparatus under the following measurement condition.

Measuring apparatus: "HLC-8320 GPC" manufactured by Tosoh Corporation
Column: Guard column "HXL-L" manufactured by Tosoh Corporation

+ "TSK-GEL G2000HXL" manufactured by Tosoh Corporation
+ "TSK-GEL G2000HXL" manufactured by Tosoh Corporation
+ "TSK-GEL G3000HXL" manufactured by Tosoh Corporation
+ "TSK-GEL G4000HXL" manufactured by Tosoh Corporation

Detector: RI (differential refractometer)
Data processing: "GPC Workstation EcoSEC-Workstation" manufactured by Tosoh Corporation
Measurement condition: Column temperature: 40°C

Developing solvent: tetrahydrofuran
Flow rate: 1.0 mL/min

Standard: the following monodisperse polystyrene having a known molecular weight was used in accordance with a measurement manual of the above-mentioned "GPC Workstation EcoSEC-Workstation".

(Used polystyrene)

"A-500" manufactured by Tosoh Corporation
"A-1000" manufactured by Tosoh Corporation
"A-2500" manufactured by Tosoh Corporation
"A-5000" manufactured by Tosoh Corporation
"F-1" manufactured by Tosoh Corporation
"F-2" manufactured by Tosoh Corporation
"F-4" manufactured by Tosoh Corporation

"F-10" manufactured by Tosoh Corporation
"F-20" manufactured by Tosoh Corporation
"F-40" manufactured by Tosoh Corporation
"F-80" manufactured by Tosoh Corporation
"F-128" manufactured by Tosoh Corporation
Sample: a sample (50 μL) obtained by filtration of tetrahydrofuran solution of the phenolic resin and the epoxy resin in an amount of 1.0% by mass in terms of solid content through a microfilter.

The content ratio (area%) of a residual (unreacted) catechol compound and a residual (unreacted) phenol compound in the phenolic resin is preferably 1 area% or less, more preferably 0.8 area% or less, and even more preferably 0.4 area% or less, as the total content ratio of the residual catechol compound (residual catechol) and the residual phenol compound (residual phenol) from the viewpoint of decreasing the volatile content during curing.

<FD-MS Spectrum Measurement>

An FD-MS spectrum was measured with the following measurement apparatus under the following measurement condition.
Measuring apparatus: JMS-T100GC AccuTOF manufactured by JEOL Ltd.

Measuring conditions

Measurement range: m/z = 4.00 to 2000.00
Change rate: 51.2 mA/min
Final electric current: 45 mA
Cathode voltage: -10 kV
Record interval: 0.07 sec

From the results of the FD-MS spectrum, peaks derived from targeted products (phenolic resin and epoxy resin) were confirmed, indicating the acquisition of the targeted products. The measurement results of the FD-MS spectrum only in Example 1 (see FIG. 15) and Example 8 (see FIG. 16) are shown.

<$^{13}$C-NMR Measurement>

$^{13}$C-NMR: "JNM-ECA500" manufactured by JEOL Ltd.
Magnetic field intensity: 500 MHz
Integration count: 2,000 times
Solvent: DMSO-d6 (phenolic resin), chloroform-d1 (epoxy resin)
Sample concentration: 30% by mass

From the results of the $^{13}$C-NMR chart, peaks derived from targeted products (phenolic resin and epoxy resin) were confirmed, indicating the acquisition of the targeted products in reactions. The measurement results of the $^{13}$C-NMR ($^{13}$C-NMR chart) only in Example 1 (see FIG. 17) and Example 8 (see FIG. 18) are shown.

Example 1

[Synthesis of Phenolic Resin (a-1)]

In a 2-L flask equipped with a thermometer, a condenser, and a stirrer, 484.4 g (4.40 mol) of catechol, 414.0 g (4.40 mol) of phenol, and 154.2 g (0.88 mol) of o-xylylene dichloride were placed, heated to 100°C with dissolving, and reacted while the temperature was held for 3 hours. Subsequently, the temperature was increased to 150°C, and a reaction was allowed to proceed while the temperature was held for 3 hours. At that time, hydrogen chloride produced by the reaction was discharged to the outside of the system, and absorbed to a trap of an aqueous sodium hydroxide solution. After the reaction, unreacted catechol and phenol were removed under reduced pressure, to obtain 236.4 g of phenolic resin (a-1). The hydroxy group equivalent was 96 g/eq, the softening point was 63°C, and the melt viscosity at 150°C was

0.4 dPa·s. The GPC area percentage of the residual catechol (see FIG. 1) was 0.2 area%, and the phenol was not detected.

Example 2

[Synthesis of Phenolic Resin (a-2)]

239.4 g of phenolic resin (a-2) was obtained by the same operation as in Example 1 except that the amount of catechol was changed to 387.6 g (3.52 mol), and the amount of phenol was changed to 496.8 g (5.28 mol). The hydroxy group equivalent was 104 g/eq, the softening point was 62°C, and the melt viscosity at 150°C was 0.4 dPa·s. Regarding GPC (see FIG. 2), the residual catechol and the residual phenol were not detected.

Example 3

[Synthesis of Phenolic Resin (a-3)]

245.4 g of phenolic resin (a-3) was obtained by the same operation as in Example 1 except that the amount of catechol was changed to 290.7 g (2.64 mol), and the amount of phenol was changed to 579.7 g (6.16 mol). The hydroxy group equivalent was 107 g/eq, the softening point was 61°C, and the melt viscosity at 150°C was 0.3 dPa·s. The GPC area percentage of the residual catechol (see FIG. 3) was 0.3 area%, and the phenol was not detected.

Example 4

[Synthesis of Phenolic Resin (a-4)]

241.9 g of phenolic resin (a-4) was obtained by the same operation as in Example 1 except that the amount of catechol was changed to 193.8 g (1.76 mol), and the amount of phenol was changed to 662.5 g (7.04 mol). The hydroxy group equivalent was 120 g/eq, the softening point was 57°C, and the melt viscosity at 150°C was 0.3 dPa·s. The GPC area percentage of the residual catechol (see FIG. 4) was 0.5 area%, and the phenol was not detected.

Example 5

[Synthesis of Phenolic Resin (a-5)]

229.1 g of phenolic resin (a-5) was obtained by the same operation as in Example 1 except that the amount of catechol was changed to 96.9 g (0.88 mol), and the amount of phenol was changed to 745.3 g (7.92 mol). The hydroxy group equivalent was 134 g/eq, the softening point was 53°C, and the melt viscosity at 150°C was 0.2 dPa·s. Regarding GPC (see FIG. 5), the residual catechol and the residual phenol were not detected.

Example 6

[Synthesis of Phenolic Resin (a-6)]

In a 2-L flask equipped with a thermometer, a condenser, and a stirrer, 264.2 g (2.40 mol) of catechol, 338.8 g (3.60 mol) of phenol, and 210.1 g (1.20 mol) of o-xylylene dichloride were placed, heated to 100°C with dissolving, and reacted while the temperature was held for 3 hours. Subsequently, the temperature was increased to 150°C, and a reaction was allowed to proceed while the temperature was held for 3 hours. At that time, hydrogen chloride produced by the reaction was discharged to the outside of the system, and absorbed to a trap of an aqueous sodium hydroxide solution. After the reaction, unreacted catechol and phenol were removed under reduced pressure, to obtain 317.5 g of phenolic resin (a-6). The hydroxyl group equivalent was 110 g/equivalent, the softening point was 69°C, and the melt viscosity at 150°C was 0.7 dPa·s. The GPC area percentage of the residual catechol (see FIG. 6) was 0.4 area%, and the phenol was not detected.

Example 7

[Synthesis of Phenolic Resin (a-7)]

292.9 g of phenolic resin (a-7) was obtained by the same operation as in Example 5 except that the amount of

catechol was changed to 198.2 g (1.80 mol), and the amount of phenol was changed to 395.2 g (4.20 mol). The hydroxyl group equivalent was 116 g/equivalent, the softening point was 67°C, and the melt viscosity at 150°C was 0.5 dPa·s. The GPC area percentage of the residual catechol (see FIG. 7) was 0.3 area%, and the phenol was not detected.

Comparative Example 1

[Synthesis of Phenolic Resin (b-1)]

In a 2-L flask equipped with a thermometer, a condenser, and a stirrer, 968.9 g (8.80 mol) of catechol, 154.2 g (0.88 mol) of o-xylylene dichloride, and 96.9 g of methanol were placed, heated to 120°C with dissolving, and reacted while the temperature was held for 3 hours. Subsequently, the temperature was increased to 150°C, and a reaction was allowed to proceed while the temperature was held for 3 hours. At that time, hydrogen chloride produced by the reaction was discharged to the outside of the system, and absorbed to a trap of an aqueous sodium hydroxide solution. After the reaction, unreacted catechol was removed under reduced pressure, to obtain 228.2 g of phenolic resin (b-1). The hydroxyl group equivalent was 85 g/equivalent, the softening point was 70°C, and the melt viscosity at 150°C was 0.7 dPa·s. The GPC area percentage of the residual catechol (see FIG. 19) was 0.5 area%.

Comparative Example 2

[Synthesis of Phenolic Resin (b-2)]

In a 2-L flask equipped with a thermometer, a condenser, and a stirrer, 846.9 g (9.00 mol) of phenol, and 157.7 g (0.90 mol) of o-xylylene dichloride were placed, heated to 100°C with dissolving, and reacted while the temperature was held for 3 hours. Subsequently, the temperature was increased to 150°C, and a reaction was allowed to proceed while the temperature was held for 1 hour. At that time, hydrogen chloride gas produced by the reaction was discharged to the outside of the system, and absorbed to an aqueous sodium hydroxide solution. After the reaction, unreacted phenol was removed under reduced pressure, to obtain 252.1 g of phenolic resin (b-2). The hydroxy group equivalent was 152 g/eq, the softening point was 47°C, and the melt viscosity at 150°C was 0.2 dPa·s. Regarding GPC (see FIG. 20), the residual phenol was not detected.

Example 8

[Synthesis of Epoxy Resin (A-1)]

In a 2-L flask equipped with a thermometer, a condenser, and a stirrer, 192.0 g (2.0 mol in terms of hydroxy group) of the phenolic resin (a-1) and 1,110.0 g (12.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After completion of the reaction, 222.0 g of n-butanol and 260.4 g of water were added, stirring was terminated, a water layer phase collected at a lower layer was removed, stirring was restarted, and unreacted epichlorohydrin was removed under reduced pressure at 150°C. To the resultant crude epoxy resin, 516.8 g of methyl isobutyl ketone and 86.1 g of n-butanol were added and allowed to dissolve. To this solution, 17.8 g of aqueous 10% sodium hydroxide solution was added, a reaction was allowed to proceed at 80°C for 2 hours, and the resultant was repeatedly washed with 152.0 g of water until the pH of the washing liquid was neutral. Subsequently, the system was dehydrated by azeotropy, microfiltration was performed, and the solvent was removed under reduced pressure, to obtain 288.8 g of targeted epoxy resin (A-1) The epoxy equivalent of the obtained epoxy resin (A-1) was 193 g/eq, and the melt viscosity at 150°C was 0.4 dPa·s. The GPC chart is shown in FIG. 8.

Example 9

[Synthesis of Epoxy Resin (A-2)]

In a 2-L flask equipped with a thermometer, a condenser, and a stirrer, 208.0 g (2.0 mol in terms of hydroxy group) of the phenolic resin (a-2) and 1,110.0 g (12.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After

completion of the reaction, 222.0 g of n-butanol and 260.4 g of water were added, stirring was terminated, a water layer phase collected at a lower layer was removed, stirring was restarted, and unreacted epichlorohydrin was removed under reduced pressure at 150°C. To the resultant crude epoxy resin, 544.0 g of methyl isobutyl ketone and 90.7 g of n-butanol were added and allowed to dissolve. To this solution, 18.7 g of aqueous 10% sodium hydroxide solution was added, a reaction was allowed to proceed at 80°C for 2 hours, and the resultant was repeatedly washed with 160.0 g of water until the pH of the washing liquid was neutral. Subsequently, the system was dehydrated by azeotropy, microfiltration was performed, and the solvent was removed under reduced pressure, to obtain 304.0 g of targeted epoxy resin (A-2). The epoxy equivalent of the obtained epoxy resin (A-2) was 195 g/eq, and the melt viscosity at 150°C was 0.4 dPa·s. The GPC chart is shown in FIG. 9.

Example 10

[Synthesis of Epoxy Resin (A-3)]

In a 2-L flask equipped with a thermometer, a condenser, and a stirrer, 214.0 g (2.0 mol in terms of hydroxy group) of the phenolic resin (a-3) and 1,110.0 g (12.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After completion of the reaction, 222.0 g of n-butanol and 260.4 g of water were added, stirring was terminated, a water layer phase collected at a lower layer was removed, stirring was restarted, and unreacted epichlorohydrin was removed under reduced pressure at 150°C. To the resultant crude epoxy resin, 554.2 g of methyl isobutyl ketone and 92.4 g of n-butanol were added and allowed to dissolve. To this solution, 18.2 g of aqueous 10% sodium hydroxide solution was added, a reaction was allowed to proceed at 80°C for 2 hours, and the resultant was repeatedly washed with 163.0 g of water until the pH of the washing liquid was neutral. Subsequently, the system was dehydrated by azeotropy, microfiltration was performed, and the solvent was removed under reduced pressure, to obtain 308.1 g of targeted epoxy resin (A-3). The epoxy equivalent of the obtained epoxy resin (A-3) was 212 g/eq, and the melt viscosity at 150°C was 0.4 dPa·s. The GPC chart is shown in FIG. 10.

Example 11

[Synthesis of Epoxy Resin (A-4)]

In a 2-L flask equipped with a thermometer, a condenser, and a stirrer, 240.0 g (2.0 mol in terms of hydroxy group) of the phenolic resin (a-4) and 1,110.0 g (12.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After completion of the reaction, 222.0 g of n-butanol and 260.4 g of water were added, stirring was terminated, a water layer phase collected at a lower layer was removed, stirring was restarted, and unreacted epichlorohydrin was removed under reduced pressure at 150°C. To the resultant crude epoxy resin, 598.4 g of methyl isobutyl ketone and 99.7 g of n-butanol were added and allowed to dissolve. To this solution, 15.3 g of aqueous 10% sodium hydroxide solution was added, a reaction was allowed to proceed at 80°C for 2 hours, and the resultant was repeatedly washed with 176.0 g of water until the pH of the washing liquid was neutral. Subsequently, the system was dehydrated by azeotropy, microfiltration was performed, and the solvent was removed under reduced pressure, to obtain 295.6 g of targeted epoxy resin (A-4). The epoxy equivalent of the obtained epoxy resin (A-4) was 225 g/eq, and the melt viscosity at 150°C was 0.3 dPa·s. The GPC chart is shown in FIG. 11.

Example 12

[Synthesis of Epoxy Resin (A-5)]

In a 2-L flask equipped with a thermometer, a condenser, and a stirrer, 214.4 g (1.6 mol in terms of hydroxy group) of the phenolic resin (a-5) and 888.8 g (9.60 mol) of epichlorohydrin were placed, and heated to 50°C with stirring and dissolving. Subsequently, 1.36 g of benzyltrimethylammonium chloride was added, and a reaction was allowed to proceed at a temperature of 50°C for 24 hours. Further, 143.7 g (1.10 eq in terms of hydroxy group) of aqueous 49% sodium hydroxide solution was added dropwise over 3 hours, and a reaction was allowed to proceed at 50°C for 1 hour. After completion of the reaction, 177.8 g of n-butanol and 208.4 g of water were added, stirring was terminated, a water layer

phase collected at a lower layer was removed, stirring was restarted, and unreacted epichlorohydrin was removed under reduced pressure at 150°C. To the resultant crude epoxy resin, 516.8 g of methyl isobutyl ketone and 86.1 g of n-butanol were added and allowed to dissolve. To this solution, 15.1 g of aqueous 10% sodium hydroxide solution was added, a reaction was allowed to proceed at 80°C for 2 hours, and the resultant was repeatedly washed with 152.0 g of water until the pH of the washing liquid was neutral. Subsequently, the system was dehydrated by azeotropy, microfiltration was performed, and the solvent was removed under reduced pressure, to obtain 287.8 g of targeted epoxy resin (A-5). The epoxy equivalent of the obtained epoxy resin (A-5) was 232 g/eq, and the melt viscosity at 150°C was 0.2 dPa·s. The GPC chart is shown in FIG. 12.

Example 13

[Synthesis of Epoxy Resin (A-6)]

In a 2-L flask equipped with a thermometer, a condenser, and a stirrer, 220.0 g (2.0 mol in terms of hydroxy group) of the phenolic resin (a-6) and 1,110.0 g (12.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After completion of the reaction, 222.0 g of n-butanol and 260.4 g of water were added, stirring was terminated, a water layer phase collected at a lower layer was removed, stirring was restarted, and unreacted epichlorohydrin was removed under reduced pressure at 150°C. To the resultant crude epoxy resin, 564.4 g of methyl isobutyl ketone and 94.1 g of n-butanol were added and allowed to dissolve. To this solution, 20.6 g of aqueous 10% sodium hydroxide solution was added, a reaction was allowed to proceed at 80°C for 2 hours, and the resultant was repeatedly washed with 166.0 g of water until the pH of the washing liquid was neutral. Subsequently, the system was dehydrated by azeotropy, microfiltration was performed, and the solvent was removed under reduced pressure, to obtain 295.6 g of targeted epoxy resin (A-6). The epoxy equivalent of the obtained epoxy resin (A-6) was 203 g/eq, and the melt viscosity at 150°C was 0.7 dPa·s. The GPC chart is shown in FIG. 13.

Example 14

[Synthesis of Epoxy Resin (A-7)]

In a 2-L flask equipped with a thermometer, a condenser, and a stirrer, 232.0 g (2.0 mol in terms of hydroxy group) of the phenolic resin (a-7) and 1,110.0 g (12.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After completion of the reaction, 222.0 g of n-butanol and 260.4 g of water were added, stirring was terminated, a water layer phase collected at a lower layer was removed, stirring was restarted, and unreacted epichlorohydrin was removed under reduced pressure at 150°C. To the resultant crude epoxy resin, 584.8 g of methyl isobutyl ketone and 94.1 g of n-butanol were added and allowed to dissolve. To this solution, 20.6 g of aqueous 10% sodium hydroxide solution was added, a reaction was allowed to proceed at 80°C for 2 hours, and the resultant was repeatedly washed with 172.0 g of water until the pH of the washing liquid was neutral. Subsequently, the system was dehydrated by azeotropy, microfiltration was performed, and the solvent was removed under reduced pressure, to obtain 295.6 g of targeted epoxy resin (A-7). The epoxy equivalent of the obtained epoxy resin (A-7) was 222 g/eq, and the melt viscosity at 150°C was 0.7 dPa·s. The GPC chart is shown in FIG. 14.

Comparative Example 3

[Synthesis of Epoxy Resin (B-1)]

In a 2-L flask equipped with a thermometer, a condenser, and a stirrer, 170.0 g (2.0 mol in terms of hydroxy group) of the phenolic resin (b-1) and 740.0 g (8.0 mol) of epichlorohydrin were placed, and heated to 50°C with stirring and dissolving. Subsequently, 1.70 g of benzyltrimethylammonium chloride was added, and a reaction was allowed to proceed at a temperature of 50°C for 24 hours. Further, 179.6 g (1.10 eq in terms of hydroxy group) of aqueous 49% sodium hydroxide solution was added dropwise over 3 hours, and a reaction was allowed to proceed at 50°C for 1 hour. After completion of the reaction, 148.0 g of n-butanol and 260.4 g of water were added, stirring was terminated, a water layer phase collected at a lower layer was removed, stirring was restarted, and unreacted epichlorohydrin was removed under

reduced pressure at 150°C. To the resultant crude epoxy resin, 489.6 g of methyl isobutyl ketone and 81.6 g of n-butanol were added and allowed to dissolve. To this solution, 21.1 g of aqueous 10% sodium hydroxide solution was added, a reaction was allowed to proceed at 80°C for 2 hours, and the resultant was repeatedly washed with 144.0 g of water until the pH of the washing liquid was neutral. Subsequently, the system was dehydrated by azeotropy, microfiltration was performed, and the solvent was removed under reduced pressure, to obtain 272.0 g of targeted epoxy resin (B-1). The epoxy equivalent of the obtained epoxy resin (B-1) was 164 g/eq, and the melt viscosity at 150°C was 0.4 dPa·s. The GPC chart is shown in FIG. 21.

Comparative Example 4

[Synthesis of Epoxy Resin (B-2)]

In a 2-L flask equipped with a thermometer, a condenser, and a stirrer, 243.2 g (1.60 mol in terms of hydroxy group) of the phenolic resin (b-2), 740.0 g (8.00 mol) of epichlorohydrin, and 148.0 g of n-butanol were placed, and heated to 50°C with stirring and dissolving. Subsequently, 143.7 g (1.10 eq in terms of hydroxy group) of aqueous 49% sodium hydroxide solution was added dropwise at a temperature of 50°C over 3 hours, and a reaction was allowed to proceed for 1 hour. After completion of the reaction, 148.0 g of n-butanol and 208.4 g of water were added, stirring was terminated, a water layer phase collected at a lower layer was removed, stirring was restarted, and unreacted epichlorohydrin was removed under reduced pressure at 150°C. To the resultant crude epoxy resin, 565.8 g of methyl isobutyl ketone and 94.3 g of n-butanol were added and allowed to dissolve. To this solution, 11.2 g of aqueous 10% sodium hydroxide solution was added, a reaction was allowed to proceed at 80°C for 2 hours, and the resultant was repeatedly washed with 166.4 g of water until the pH of the washing liquid was neutral. Subsequently, the system was dehydrated by azeotropy, microfiltration was performed, and the solvent was removed under reduced pressure, to obtain 314.5 g of targeted epoxy resin (B-2). The epoxy equivalent of the obtained epoxy resin (B-2) was 217 g/eq, and the melt viscosity at 25°C was 304,000 mPa·s. The GPC chart is shown in FIG. 22.

Comparative Example 5

[Synthesis of Epoxy Resin (B-3)]

A bisphenol A type liquid epoxy resin, EPICLON 850S manufactured by DIC Corporation (epoxy equivalent: 188 g/eq, viscosity at 25°C: 13,000 mPa·s) was used as an epoxy resin (B-3) .

Comparative Example 6

[Synthesis of Epoxy Resin (B-4)]

A tetraglycidyl diaminodiphenylmethane type epoxy resin, SUMI-EPOXY ELM-434 manufactured by Sumitomo Chemical Co., Ltd. (epoxy equivalent: 121 g/eq, viscosity at 25°C: 388,000 mPa·s) was used as an epoxy resin (B-4).

Examples 15 to 21 and Comparative Examples 7 to 10

The epoxy resin in each of Examples 8 to 14 and Comparative Examples 3 to 6 and 4,4'-diaminodiphenylsulfone (4,4'-DDS) as a curing agent were supplied at a ratio of epoxy equivalent to active hydrogen equivalent of 1 to 1, and melt-mixed at 100 to 120°C, to obtain an epoxy resin composition. The epoxy resin composition was placed in a space between glass plates between which a 4-mm spacer was disposed, and subjected to a curing reaction at 150°C for 1 hour and then at 180°C for 3 hours, to produce a cured product.

<Measurement of Bending Strength, Bending Elastic Modulus, and Bending Strain>

The bending strength, bending elastic modulus, and bending strain of the obtained cured product having a thickness of 4 mm at an early stage and after water absorption by 2-week immersion in water at 70°C were each measured in accordance with JIS K 7171.

The bending elastic modulus is preferably 3,000 MPa or more, more preferably 3,200 MPa or more, and even more preferably 3,400 MPa or more.

The bending strength is preferably 130 MPa or more, more preferably 140 MPa or more, and even more preferably 150 MPa or more.

The bending strain is preferably 4.0% or more, more preferably 4.5% or more, and even more preferably 5% or

more.

When the values of the bending properties at the early stage and after water absorption are within the aforementioned ranges, excellent high bending properties can be expressed, and the cured product is useful.

<Measurement of Water Absorption Ratio>

The resultant cured product was immersed in water at 70°C for two weeks, and then taken from water, and the water absorption ratio was measured. The water absorption ratio was calculated by the following equation.

Water absorption ratio= 100 × [(weight of cured product after water absorption) - (weight of cured product at early stage)]/[weight of cured product at early stage)]

The water absorption ratio is preferably 3% or less, more preferably 2.5% or less, and even more preferably 2% or less.

[Table 1]

| Physical values of phenolic resin | | Example | | | | | | | Comparative Example | |
|---|---|---|---|---|---|---|---|---|---|---|
| | | 1 | 2 | 3 | 4 | 5 | 6 | 7 | 1 | 2 |
| Phenolic resin | | (a-1) | (a-2) | (a-3) | (a-4) | (a-5) | (a-6) | (a-7) | (b-1) | (b-2) |
| Hydroxy group equivalent | g/eq. | 96 | 104 | 107 | 120 | 134 | 110 | 116 | 85 | 152 |
| Softening point | °C | 63 | 62 | 61 | 57 | 53 | 69 | 67 | 70 | 47 |
| Melt viscosity at 150°C | dPa·s | 0.4 | 0.4 | 0.3 | 0.3 | 0.2 | 0.7 | 0.5 | 0.7 | 0.2 |
| Residual catecol | area% | 0.2 | Not detected | 0.3 | 0.5 | Not detected | 0.4 | 0.3 | 0.5 | - |
| Residual phenol | area% | Not detected | Not detected | Not detected | Not detected | Not detected | Not detected | Not detected | - | Not detected |

[Table 2]

| Physical values of epoxy resin | | Example | | | | | | | Comparative Example | | | |
|---|---|---|---|---|---|---|---|---|---|---|---|---|
| | | 8 | 9 | 10 | 11 | 12 | 13 | 14 | 3 | 4 | 5 | 6 |
| Epoxy resin | | (A-1) | (A-2) | (A-3) | (A-4) | (A-5) | (A-6) | (A-7) | (B-1) | (B-2) | (B-3) | (B-4) |
| Epoxy equivalent | g/eq. | 193 | 195 | 212 | 225 | 232 | 203 | 222 | 164 | 217 | 188 | 121 |
| Melt viscosity at 150°C | dPa·s | 0.4 | 0.4 | 0.4 | 0.3 | 0.2 | 0.7 | 0.7 | 0.4 | - | - | - |
| Viscosity at 25°C | mPa·s | - | - | - | - | - | - | - | - | 304,000 | 13,000 | 388,000 |

[Table 3]

| Mixed contents (unit: g) | | Example | | | | | | | Comparative Example | | | |
|---|---|---|---|---|---|---|---|---|---|---|---|---|
| | | 15 | 16 | 17 | 18 | 19 | 20 | 21 | 7 | 8 | 9 | 10 |
| Epoxy resin | (A-1) | 100 | | | | | | | | | | |
| | (A-2) | | 100 | | | | | | | | | |
| | (A-3) | | | 100 | | | | | | | | |
| | (A-4) | | | | 100 | | | | | | | |
| | (A-5) | | | | | 100 | | | | | | |
| | (A-6) | | | | | | 100 | | | | | |
| | (A-7) | | | | | | | 100 | | | | |
| | (B-1) | | | | | | | | 100 | | | |
| | (B-2) | | | | | | | | | 100 | | |
| | (B- | | | | | | | | | | 100 | |
| | 3) | | | | | | | | | | | |
| | (B-4) | | | | | | | | | | | 100 |
| 4,4'-DDS | | 32.1 | 31.8 | 29.2 | 27.6 | 26.7 | 30.5 | 27.9 | 37.8 | 28.6 | 33.0 | 51.2 |

[Table 4]

| Physical properties | | Unit | Example | | | | | | | Comparative Example | | | |
|---|---|---|---|---|---|---|---|---|---|---|---|---|---|
| | | | 15 | 16 | 17 | 18 | 19 | 20 | 21 | 7 | 8 | 9 | 10 |
| Early stage | Bending elastic modulus | MPa | 4200 | 4210 | 4140 | 4170 | 4040 | 4030 | 3990 | 3970 | 3850 | 2860 | 3600 |
| | Bending strength | MPa | 175 | 172 | 176 | 163 | 156 | 159 | 170 | 144 | 166 | 128 | 124 |
| | Bending strain | % | 5.8 | 5.7 | 6.3 | 5.6 | 4.8 | 5.8 | 6.5 | 4.7 | 9.2 | 8.7 | 4.3 |

(continued)

| Physical properties | | Unit | Example | | | | | | | Comparative Example | | | |
|---|---|---|---|---|---|---|---|---|---|---|---|---|---|
| | | | 15 | 16 | 17 | 18 | 19 | 20 | 21 | 7 | 8 | 9 | 10 |
| After water absorption | Water absorption ratio | % | 2.11 | 1.95 | 1.72 | 1.66 | 1.49 | 2.05 | 1.91 | 2.84 | 1.32 | 2.48 | 3.58 |
| | Bending elastic modulus | MPa | 3850 | 3810 | 3900 | 3980 | 3930 | 3710 | 3770 | 3730 | 3650 | 2710 | 3200 |
| | Bending strength | MPa | 139 | 138 | 146 | 155 | 156 | 142 | 144 | 122 | 128 | 114 | 94 |
| | Bending strain | % | 4.7 | 4.5 | 4 . 9 | 5.6 | 6.0 | 5.1 | 5.0 | 4.0 | 8.3 | 8.0 | 3.3 |

As indicated in Tables 1 to 4 above, the desired phenolic resins (a-1) to (a-7) (Examples 1 to 7) had low melt viscosity and excellent handlingability, the epoxy resins (A-1) to (A-7) obtained using the phenolic resins (Examples 8 to 14) had also low melt viscosity and excellent handlingability, the cured products obtained using the epoxy resins (Examples 15 to 21) had low water absorption properties and high bending properties.

On the other hand, all the bending properties in Comparative Examples did not satisfy desired ranges unlike Examples, and in particular, the bending strengths after water absorption in all Comparative Examples were lower than those in Examples.

Brief Description of Drawings

FIG. 1 is a GPC chart of the phenolic resin (a-1) obtained in Example 1.
FIG. 2 is a GPC chart of the phenolic resin (a-2) obtained in Example 2.
FIG. 3 is a GPC chart of the phenolic resin (a-3) obtained in Example 3.
FIG. 4 is a GPC chart of the phenolic resin (a-4) obtained in Example 4.
FIG. 5 is a GPC chart of the phenolic resin (a-5) obtained in Example 5.
FIG. 6 is a GPC chart of the phenolic resin (a-6) obtained in Example 6.
FIG. 7 is a GPC chart of the phenolic resin (a-7) obtained in Example 7.
FIG. 8 is a GPC chart of the epoxy resin (A-1) obtained in Example 8.
FIG. 9 is a GPC chart of the epoxy resin (A-2) obtained in Example 9.
FIG. 10 is a GPC chart of the epoxy resin (A-3) obtained in Example 10.
FIG. 11 is a GPC chart of the epoxy resin (A-4) obtained in Example 11.
FIG. 12 is a GPC chart of the epoxy resin (A-5) obtained in Example 12.
FIG. 13 is a GPC chart of the epoxy resin (A-6) obtained in Example 13.
FIG. 14 is a GPC chart of the epoxy resin (A-7) obtained in Example 14.
FIG. 15 is an FD-MS spectrum chart of the phenolic resin (a-1) obtained in Example 1.
FIG. 16 is an FD-MS spectrum chart of the epoxy resin (A-1) obtained in Example 8.
FIG. 17 is a $^{13}$C-NMR chart of the phenolic resin (a-1) obtained in Example 1.
FIG. 18 is a $^{13}$C-NMR chart of the epoxy resin (A-1) obtained in Example 8.
FIG. 19 is a GPC chart of the phenolic resin (b-1) obtained in Comparative Example 1.
FIG. 20 is a GPC chart of the phenolic resin (b-2) obtained in Comparative Example 2.
FIG. 21 is a GPC chart of the epoxy resin (B-1) obtained in Comparative Example 3.
FIG. 22 is a GPC chart of the epoxy resin (B-2) obtained in Comparative Example 4.

**Claims**

**1.**

A phenolic resin that is a reaction product of a catechol compound, a phenol compound, and an o-xylylene

skeleton-containing compound,
the phenolic resin comprising a catechol skeleton derived from the catechol compound, a phenol skeleton derived from the phenol compound, and an o-xylylene skeleton derived from the o-xylylene skeleton-containing compound.

2. The phenolic resin according to claim 1, wherein the phenolic resin is represented by a general formula (1) below:

[Formula 1]

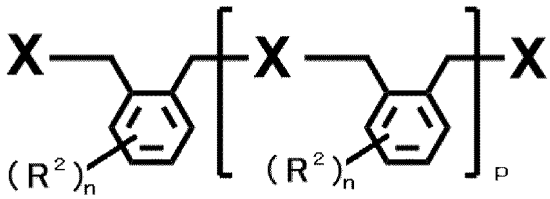

(1)

[Formula 2]

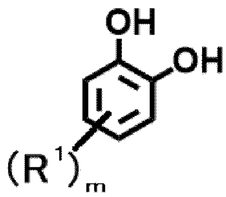

(2)

[Formula 3]

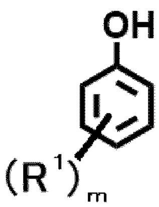

(3)

wherein X is a catechol compound represented by a formula (2), or a phenol compound represented by a formula (3), $R^1$ is a hydrogen atom, a hydrocarbon group having 1 to 4 carbon atoms, or an alkoxy group having 1 to 4 carbon atoms, $R^2$ is a hydrogen atom or a methyl group, m is an integer of 0 to 3, n is an integer of 0 to 4, and p is an integer of 0 to 50.

3. The phenolic resin according to claim 1 or 2, wherein the phenolic resin has a hydroxy group equivalent of 90 to 140 g/eq.

4. An epoxy resin that is a reaction product comprising a glycidyl ether group obtained by a reaction of a phenolic hydroxy group of the phenolic resin according to any one of claims 1 to 3 with epihalohydrin.

5. The epoxy resin according to claim 4, wherein the epoxy resin is represented by a general formula (4) below:

[Formula 4]

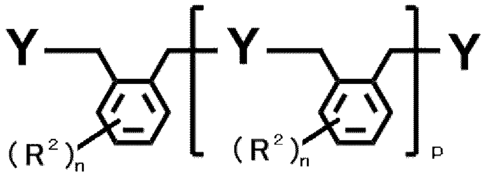

(4)

[Formula 5]

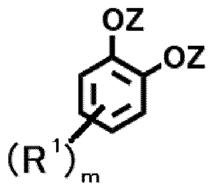

(5)

[Formula 6]

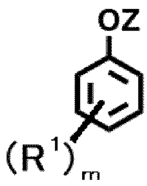

(6)

[Formula 7]

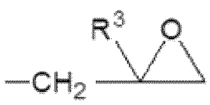

(7)

wherein Y is represented by a formula (5) or (6), Z is represented by a formula (7), $R^1$ is a hydrogen atom, a hydrocarbon group having 1 to 4 carbon atoms, or an alkoxy group having 1 to 4 carbon atoms, $R^2$ is a hydrogen atom or a methyl group, $R^3$ is a hydrogen atom or a methyl group, m is an integer of 0 to 3, n is an integer of 0 to 4, and p is an integer of 0 to 50.

**6.** The epoxy resin according to claim 4 or 5, wherein the epoxy resin has an epoxy equivalent of 150 to 300 g/eq.

**7.** A curable resin composition comprising the epoxy resin according to any one of claims 4 to 6.

**8.** A cured product obtained by a curing reaction of the curable resin composition according to claim 7.

**9.** A fiber-reinforced composite material comprising the curable resin composition according to claim 7 and reinforcement fibers.

**10.** A fiber-reinforced resin molded article comprising the cured product according to claim 8 and reinforcement fibers.

FIG. 1

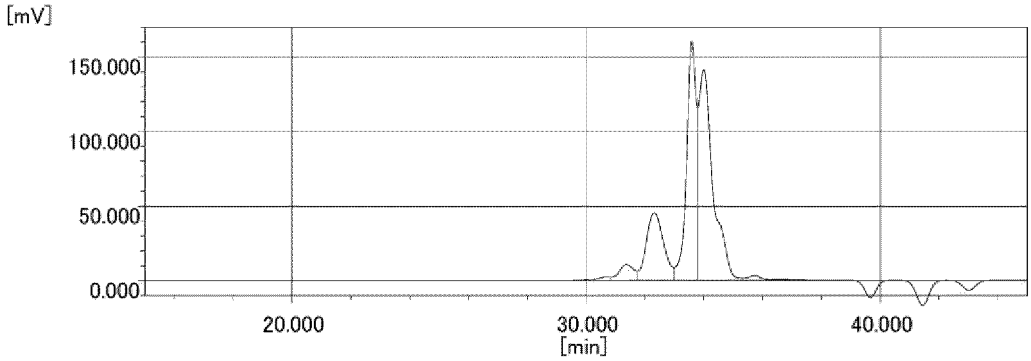


FIG. 2

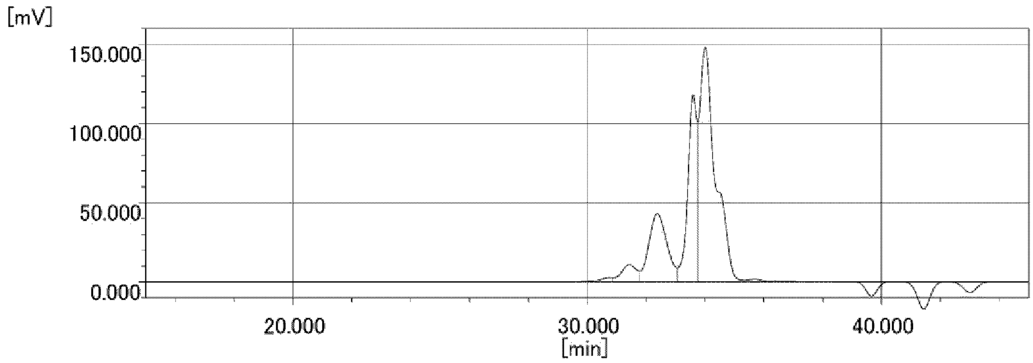


FIG. 3

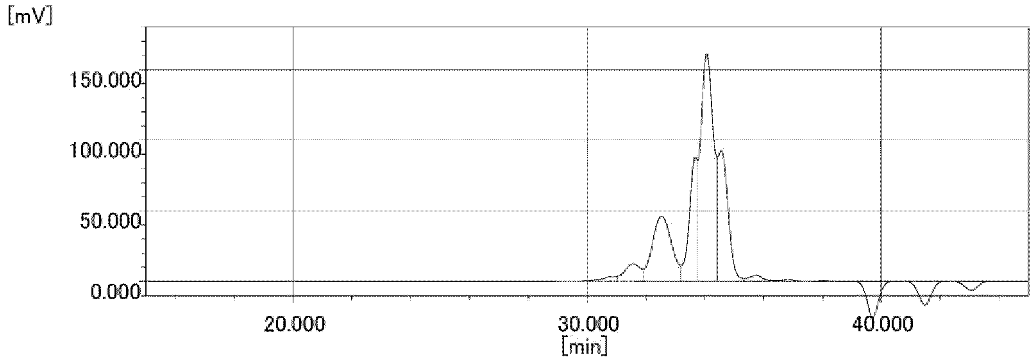

FIG. 4

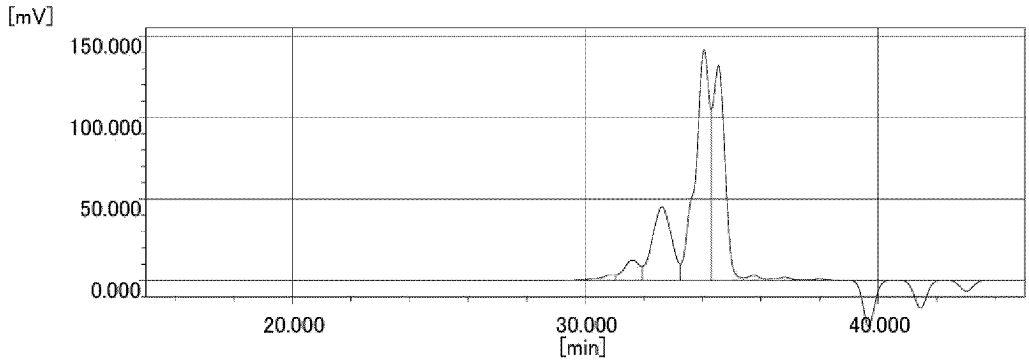


FIG. 5

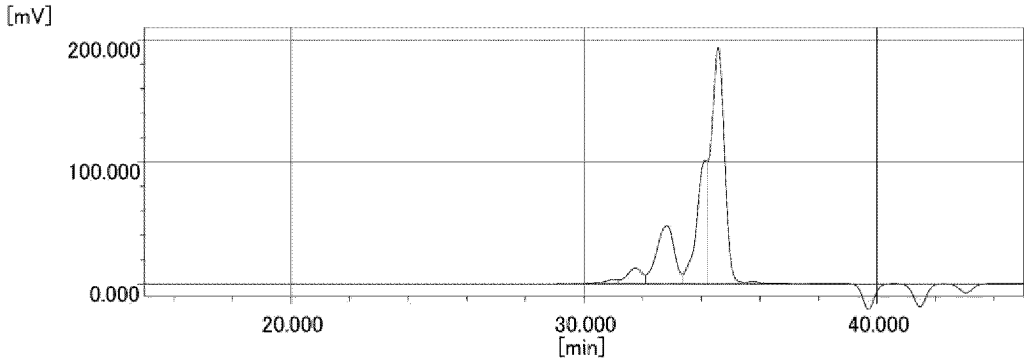


FIG. 6

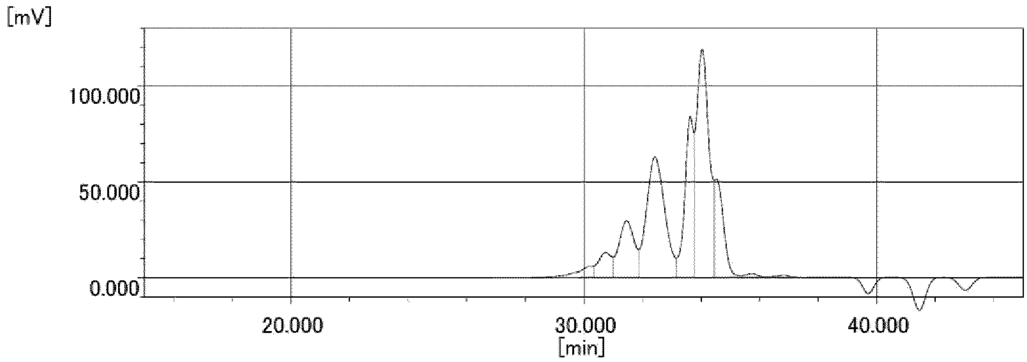

FIG. 7

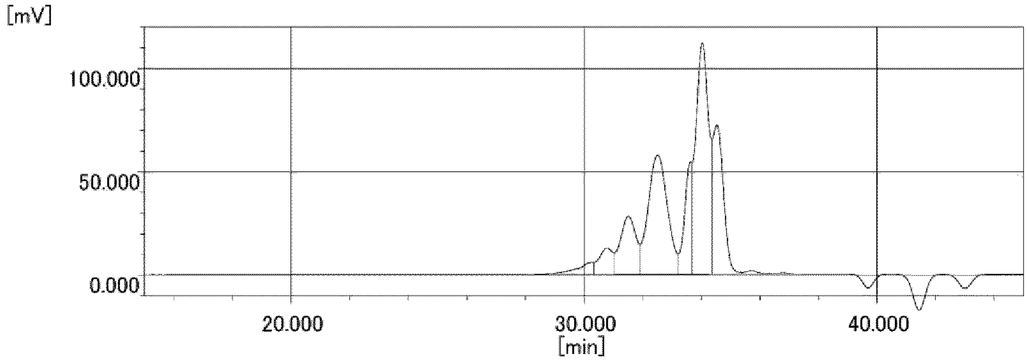


FIG. 8

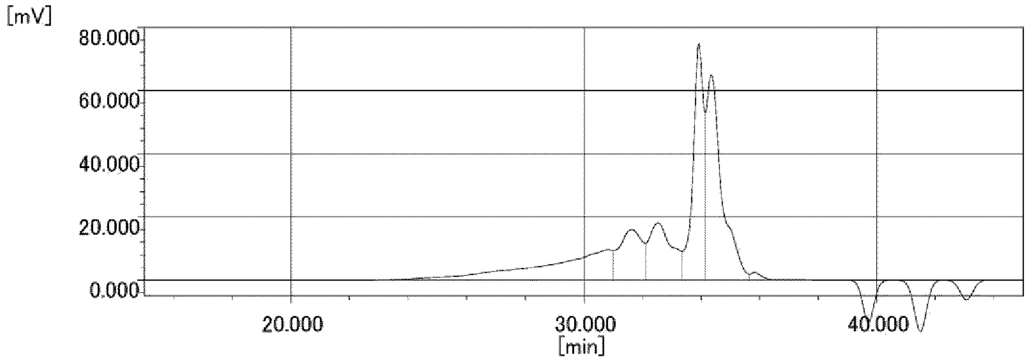


FIG. 9

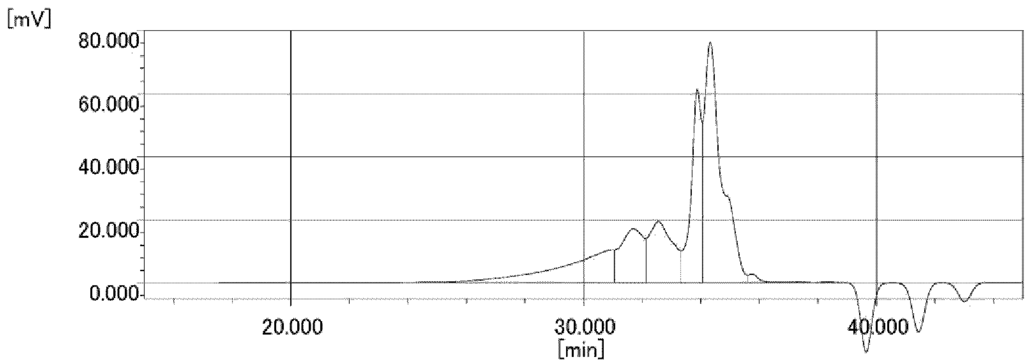

FIG. 10

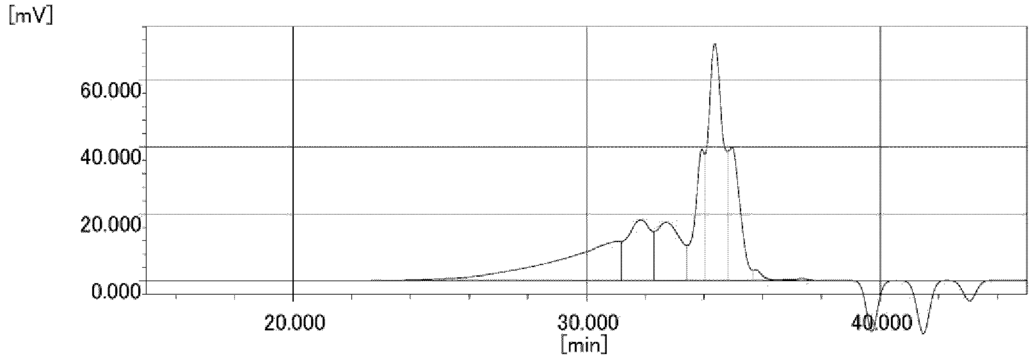


FIG. 11

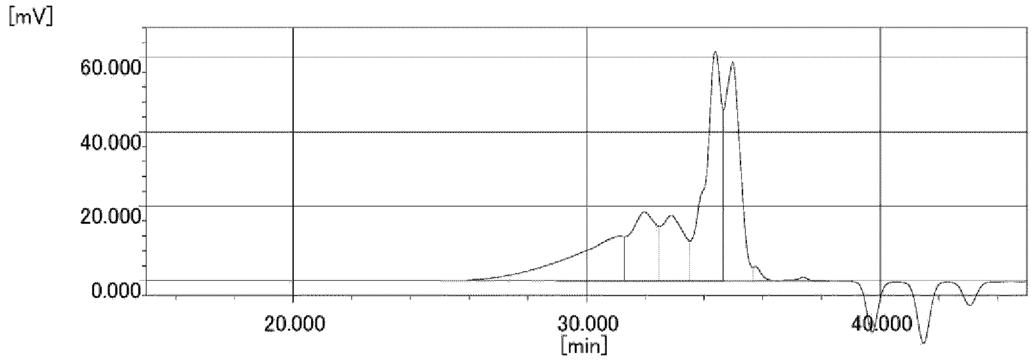


FIG. 12

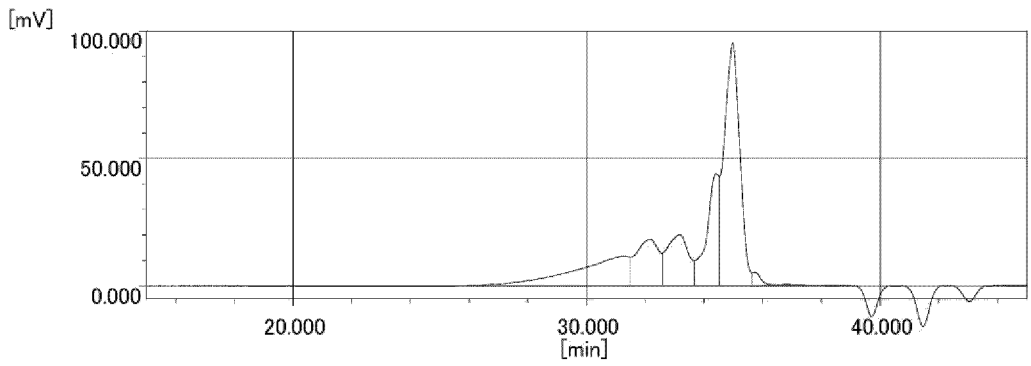

FIG. 13

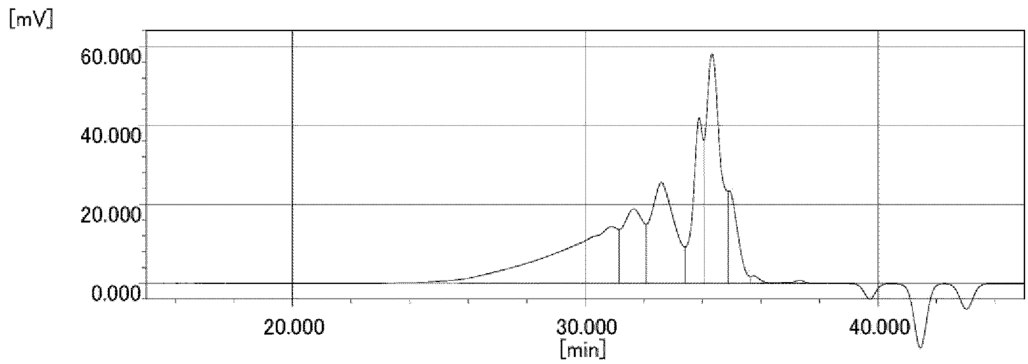
[mV]
60.000
40.000
20.000
0.000
20.000
30.000
[min]
40.000

FIG. 14

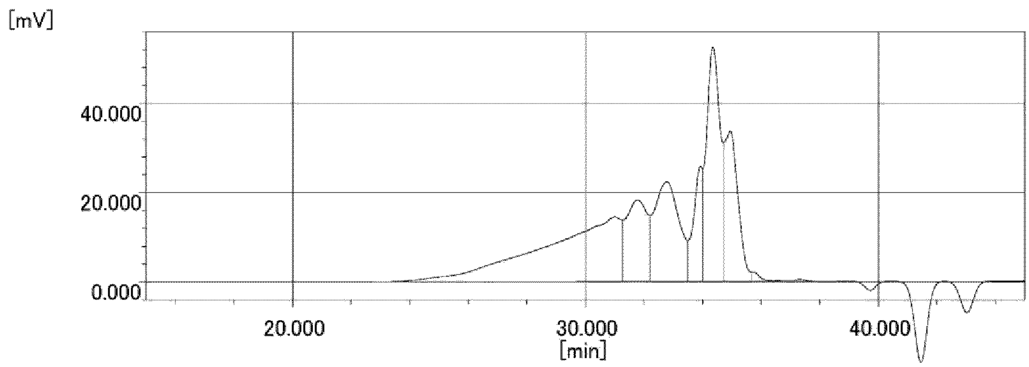
[mV]
40.000
20.000
0.000
20.000
30.000
[min]
40.000

FIG. 15

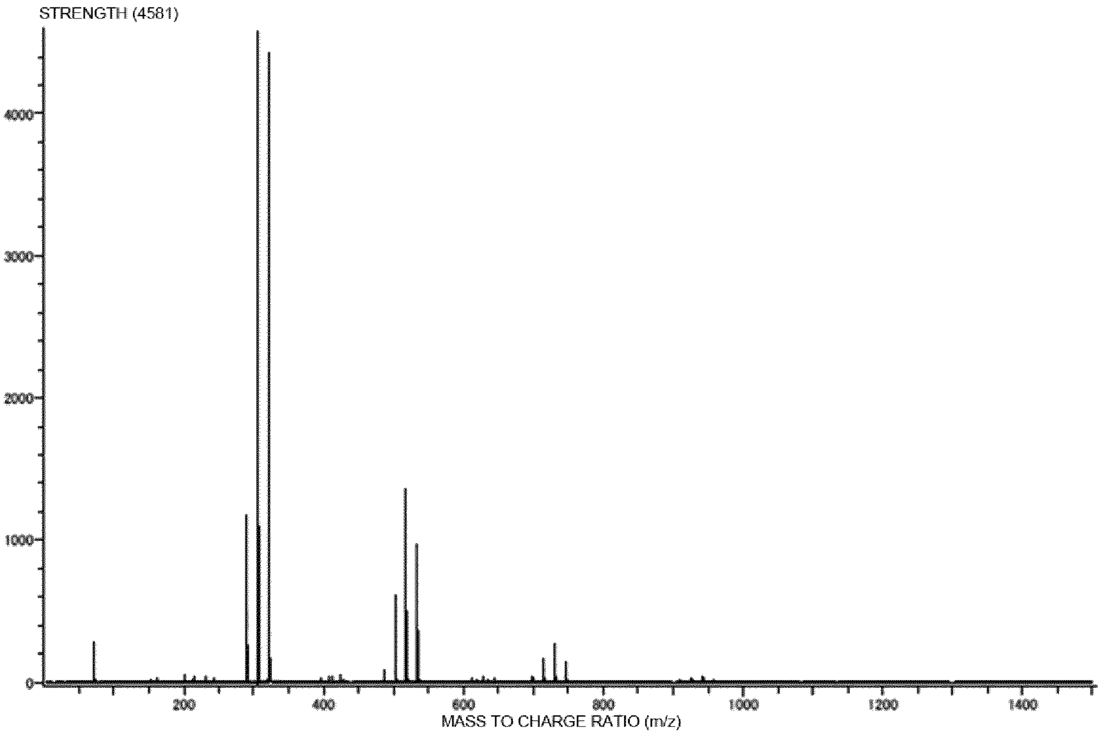
STRENGTH (4581)
4000
3000
2000
1000
0
200
400
600
800
1000
1200
1400
MASS TO CHARGE RATIO (m/z)

FIG. 16

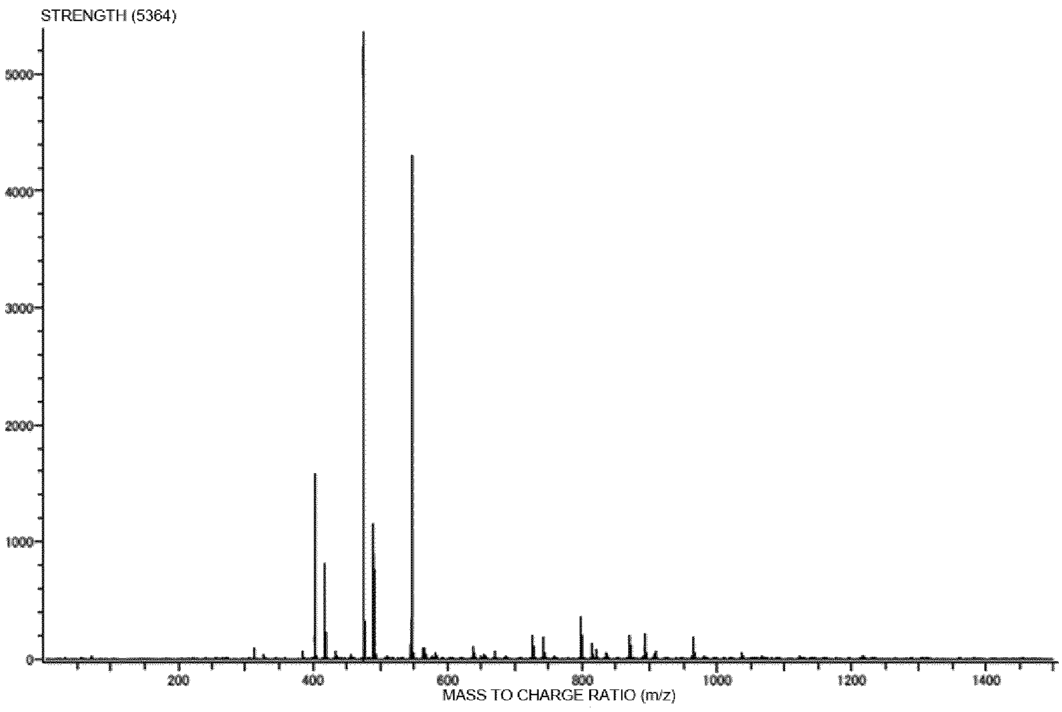
STRENGTH (5364)
5000
4000
3000
2000
1000
0
200
400
600
800
1000
1200
1400
MASS TO CHARGE RATIO (m/z)

FIG. 17

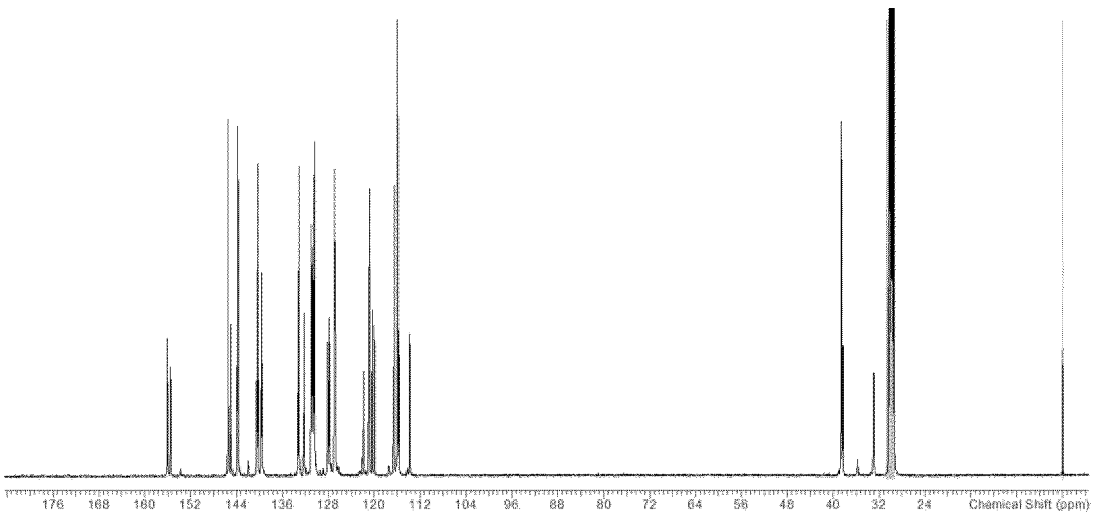
176
168
160
152
144
136
128
120
112
104
96
88
80
72
64
56
48
40
32
24
Chemical Shift (ppm)

FIG. 18

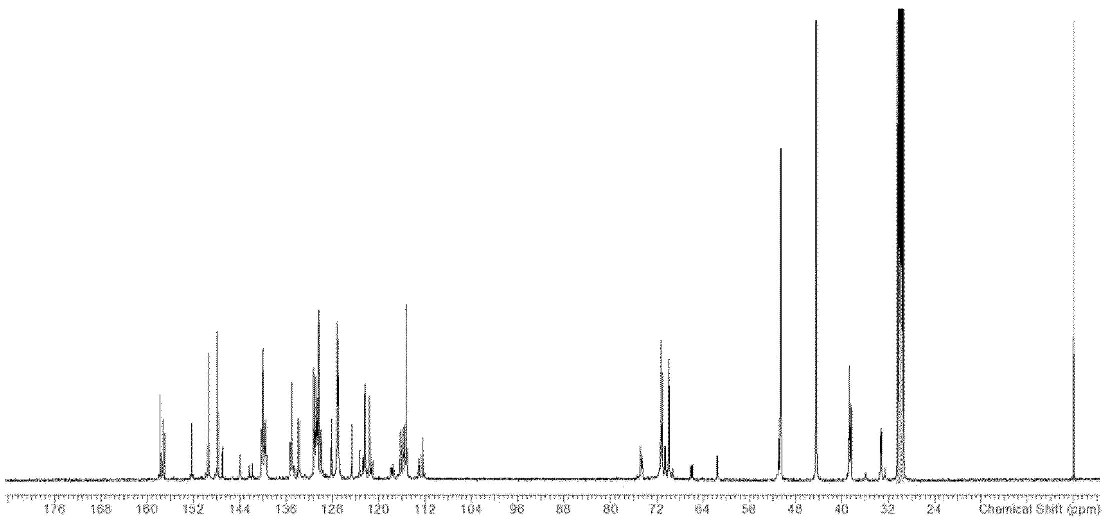
176
168
160
152
144
136
128
120
112
104
96
88
80
72
64
56
48
40
32
24
Chemical Shift (ppm)

FIG. 19

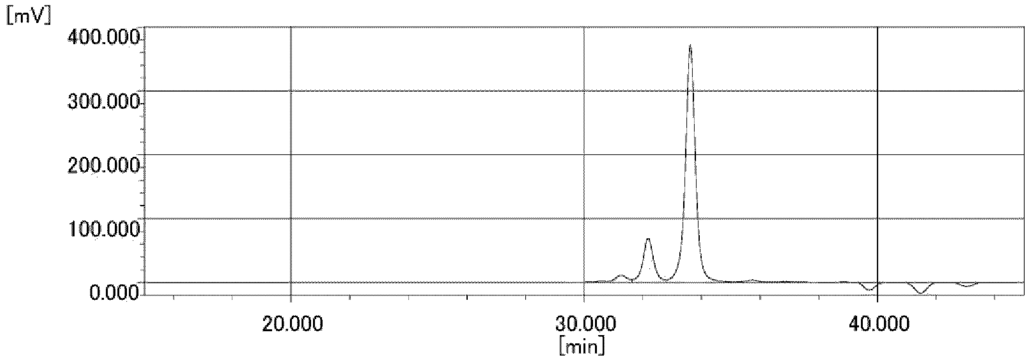


FIG. 20

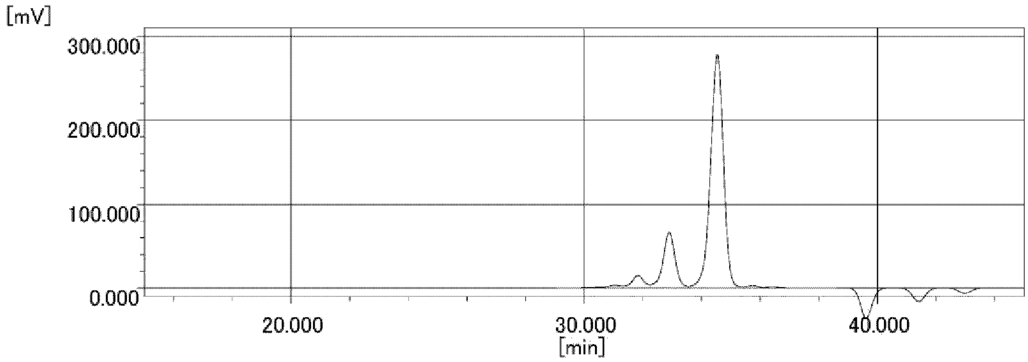


FIG. 21

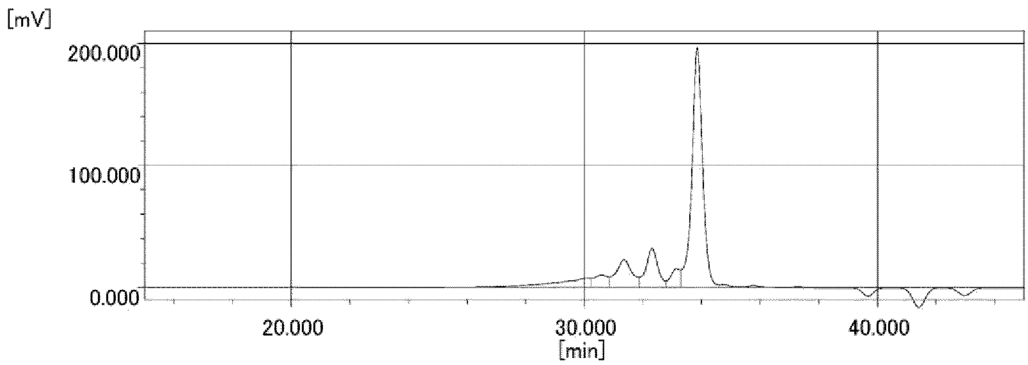

FIG. 22

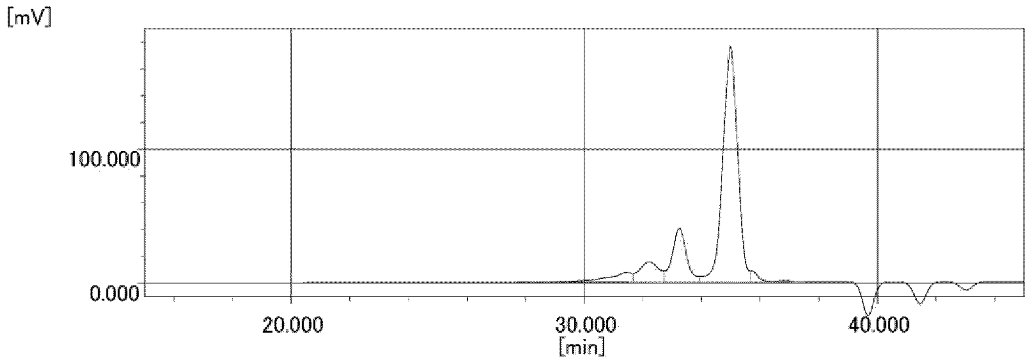

**INTERNATIONAL SEARCH REPORT**

International application No.
**PCT/JP2023/001430**

**A. CLASSIFICATION OF SUBJECT MATTER**

***C08G 61/02***(2006.01)i; ***C07D 303/48***(2006.01)i; ***C08G 59/06***(2006.01)i; ***C08G 59/20***(2006.01)i; ***C08J 5/04***(2006.01)i
FI: C08G61/02; C08G59/06; C08G59/20; C07D303/48; C08J5/04 CFC

According to International Patent Classification (IPC) or to both national classification and IPC

**B. FIELDS SEARCHED**

Minimum documentation searched (classification system followed by classification symbols)

C08G61/02; C07D303/48; C08G59/06; C08G59/20; C08J5/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2002-226559 A (NEGISHI, Akio) 14 August 2002 (2002-08-14) | 1-10 |
| A | JP 2008-189708 A (UBE IND LTD) 21 August 2008 (2008-08-21) | 1-10 |
| A | JP 2016-190891 A (NIPPON STEEL & SUMIKIN CHEM CO) 10 November 2016 (2016-11-10) | 1-10 |
| A | WO 2007/007827 A1 (UBE IND LTD) 18 January 2007 (2007-01-18) | 1-10 |
| A | JP 11-140167 A (MITSUI CHEM INC) 25 May 1999 (1999-05-25) | 1-10 |
| P, A | WO 2022/234776 A1 (DIC CORPORATION) 10 November 2022 (2022-11-10) | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**

International application No.
**PCT/JP2023/001430**

**C. DOCUMENTS CONSIDERED TO BE RELEVANT**

| Category* | Citation of document, with indication, where appropriate, of the relevant passages | Relevant to claim No. |
|---|---|---|
| P, A | JP 7103499 B1 (DIC CORPORATION) 20 July 2022 (2022-07-20) | 1-10 |

Form PCT/ISA/210 (second sheet) (January 2015)

**INTERNATIONAL SEARCH REPORT**
**Information on patent family members**

International application No.
**PCT/JP2023/001430**

| Patent document cited in search report | Publication date (day/month/year) | Patent family member(s) | Publication date (day/month/year) |
|---|---|---|---|
| JP 2002-226559 A | 14 August 2002 | (Family: none) | |
| JP 2008-189708 A | 21 August 2008 | (Family: none) | |
| JP 2016-190891 A | 10 November 2016 | (Family: none) | |
| WO 2007/007827 A1 | 18 January 2007 | CN 101208368 A<br>KR 10-2008-0030664 A<br>TW 200706613 A | |
| JP 11-140167 A | 25 May 1999 | (Family: none) | |
| WO 2022/234776 A1 | 10 November 2022 | (Family: none) | |
| JP 7103499 B1 | 20 July 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003201388 A